# EUROPEAN PATENT APPLICATION

(11) **EP 4 748 351 A1**
(43) Date of publication of application: **27.05.2026**
(21) Application number: 24844696.5
(22) Date of filing: 19.07.2024
(51) Int. Cl.: A61F 2/844, A61B 17/00, A61B 5/00

(54) **IMPLANT AND IMPLANT SYSTEM**

(30) Priority: 21.07.2023 CN 202310901178; 21.07.2023 CN 202310905176
(71) Applicant: United Innomed (Shanghai) Limited, Shanghai 201315 (CN)
(72) Inventor: WANG, Li, Shanghai 201315 (CN); GE, Shuchen, Shanghai 201315 (CN); LI, Bo, Shanghai 201315 (CN)
(74) Representative: Murgitroyd & Company
(86) International application number: PCT/CN2024/106290
(87) International publication number: WO 2025/021004

(57) **Abstract**

The present disclosure provides an implant for placement at a target site within a living body, including a therapeutic device, a wireless sensor, and a connecting member. The therapeutic device is configured to be placed at the target site to enhance the physiological functions of the living body; the wireless sensor includes a monitoring element and a wireless signal transmission element; the monitoring element is configured to acquire target physiological parameters, and the wireless signal transmission element is configured to transmit the target physiological parameters acquired by the monitoring element to the exterior of the body; wherein the connecting member is configured to connect a housing of the wireless sensor to the therapeutic device in a manner that preserves a signal transmission path of the wireless signal transmission element.

## Description

### TECHNICAL FIELD

The present disclosure relates to the field of interventional medical devices, specifically to an implant with a wireless sensor and an implant system.

### BACKGROUND

For patients with cardiovascular diseases, therapeutic devices placed in the heart or blood vessels can alleviate symptoms caused by cardiac or vascular pathologies. For instance, some patients may need to implant occluders to enhance cardiovascular function, while others may need stents to treat vascular diseases. Beyond treating cardiovascular diseases with therapeutic devices, monitoring hemodynamic parameters within the cardiovascular systems of patients is also highly significant. For instance, monitoring the atrial pressure in patients with heart failure serves as a crucial disease management tool. However, due to the factors of their small sizes, various shapes, and typically metallic composition, implants can easily interfere with wireless signals. Thus, combining monitoring devices with various therapeutic devices without introducing additional signal interference or increasing the overall size of the implants presents a major technical challenge in this field.

### SUMMARY

In view of this, the present disclosure provides an implant and an implant system for solving one or more problems in the prior art.

In the first aspect, provided is an implant for placement at a target site within a living body, comprising a therapeutic device, a wireless sensor, and a connecting member. The therapeutic device is configured to be placed at the target site to enhance the physiological function of the living body; the wireless sensor includes a monitoring element and a wireless signal transmission element; the monitoring element is configured to acquire target physiological parameters, and the wireless signal transmission element is configured to transmit the target physiological parameters acquired by the monitoring element to the exterior of the body; wherein the connecting member is configured to connect the housing of the wireless sensor to the therapeutic device in a manner that preserves the signal transmission path of the wireless signal transmission element.

In one possible implementation, the wireless sensor further includes a housing, wherein the monitoring element and the wireless signal transmission element are accommodated within the housing. The connecting member is configured to either not completely enclose the housing or to enclose the housing with a non-electromagnetic shielding structure when connected, thereby preserving the wireless signal transmission path of the wireless signal transmission element. The housing is made of a non-metallic material.

In another possible implementation, the wireless sensor further comprises a housing, with the monitoring element and the wireless signal transmission element disposed within the housing; the connecting member is provided with an accommodating cavity, the housing of the wireless sensor is provided with a mating structure adapted to the accommodating cavity, and the connecting member is sleeved onto the mating structure via the accommodating cavity.

In another possible implementation, the connecting member is provided with an insertion portion, and a housing of the wireless sensor is provided with a mating structure adapted to the insertion portion, the insertion portion is inserted into the mating structure, such that the connecting member is connected to a periphery of, or one end of the wireless sensor.

In another possible implementation, the connecting member is provided with a wireless signal transmission window, when the connecting member is sleeved onto the mating structure of the housing, the wireless signal transmission window is aligned to the wireless signal transmission element to preserve the signal transmission path; the wireless signal transmission element is further configured to receive wireless signals from the exterior of the living body.

In another possible implementation, the connecting member comprises a hanging rod and a clamping ring, wherein the clamping ring is sleeved onto the mating structure, one end of the hanging rod is connected to the clamping ring, and another end of the hanging rod is connected to the therapeutic device.

In another possible implementation, the accommodating cavity is provided on the clamping ring, the mating structure is a groove, an extending direction of the hanging rod is parallel to a central axis of the clamping ring; the connecting member further comprises an upper cover or a lower cover, and the upper cover or the lower cover is integrally formed with the clamping ring and is sleeved on one end of the housing.

In another possible implementation, the clamping ring is misaligned to the wireless signal transmission element, or, the clamping ring is aligned to the wireless signal transmission element, and the clamping ring is made of a non-metallic material, or, an opening is provided on a wall of the clamping ring, the clamping ring is a metallic tubular structure formed by helically winding a metallic strip.

In another possible implementation, the clamping ring comprises an opening and reinforcement holes, wherein the reinforcement holes are disposed at both ends of the clamping ring near the opening, for threading reinforcement members.

In another possible implementation, a groove is provided on the surface of the housing, the groove is adapted to the shape of the clamping ring, and the clamping ring is embedded in the groove.

In another possible implementation, the therapeutic device includes a mesh stent formed by cutting, the hanging rod, the clamping ring and the mesh stent are integrally formed by cutting.

In another possible implementation, the wireless sensor is hooked to the therapeutic device via the connecting member.

In another possible implementation, the therapeutic device is a valve stent, and the wireless sensor is fixed to an inflow end of, or to an outflow end of the valve stent via the connecting member.

In another possible implementation, the connecting member is connected to a wave rod of the valve stent.

In another possible implementation, when a portion of the connecting member is sleeved onto the mating structure, another portion of the connecting member is sleeved on one end of the therapeutic device.

In another possible implementation, when a portion of the connecting member is inserted into the mating structure, another portion of the connecting member is inserted into the therapeutic device.

In another possible implementation, the portion of the connecting member connected to the mating structure of the housing is an unclosed sleeve.

In another possible implementation, the therapeutic device is an occluder or a valve clip, the valve clip includes a body portion, wherein one end of the wireless sensor is inserted into the body portion via the connecting member, and the wireless signal transmission element is located outside the body portion.

In another possible implementation, the therapeutic device is an annuloplasty ring or a leadless pacemaker, and the wireless sensor is sleeved onto one end of the annuloplasty ring or the leadless pacemaker via the connecting member.

In another possible implementation, when the connecting member is sleeved onto the housing of the wireless sensor, the connecting member is configured to surround a periphery of the wireless sensor, and the therapeutic device is connected to the periphery of the wireless sensor via a lamellar structure or a filamentous structure inserted between the connecting member and the housing.

In another possible implementation, that the number of the lamellar structures or of the filamentous structures is two, and the two lamellar structures or the two filamentous structures are respectively inserted between the connecting member and the housing from opposite directions.

In another possible implementation, the connecting member comprises at least one sleeve, the sleeve is provided with an opening being configured to allow the lamellar structure or the filamentous structure to enter between the sleeve and the wireless sensor.

In another possible implementation, the connecting member includes at least one sleeve, and the sleeve is formed by clasping two semi rings.

In another possible implementation, the connecting member is a polymer sleeve, the lamellar structure or the filamentous structure is inserted between a wall of the sleeve and the wireless sensor, and is clamped by the polymer sleeve.

In another possible implementation, the therapeutic device comprises a stent formed by braiding, winding or cutting, and the stent is connected to the wireless sensor via the lamellar structure or the filamentous structure.

In another possible implementation, when the stent is connected to the wireless sensor via the filamentous structure, the filamentous structure is integrally formed with a body portion of the stent.

In another possible implementation, the therapeutic device is a chordae tendineae repair element, which includes an artificial chordae tendineae, and the artificial chordae tendineae is clamped between the connecting member and the surface of the wireless sensor.

In another possible implementation, the connecting member includes an insertion rod provided on a surface of the housing and a sleeve, the sleeve is sleeved onto the filamentous structure of the therapeutic device, and the insertion rod is inserted between the filamentous structure and the sleeve.

In another possible implementation, the connecting member is a through-hole, being provided on a wall of the housing and extending parallel to an axial direction, a filamentous structure is inserted into the through-hole, such that the therapeutic device is connected to the wireless sensor.

In another possible implementation, the number of through-holes is at least two, and the filamentous structure connects the wireless sensor to the therapeutic device by sequentially passing through at least two of the through-holes.

In another possible implementation, that the therapeutic device is a cardiac catheter pump assembly, the cardiac catheter pump assembly comprises a guide wire, wherein the guide wire forms the filamentous structure.

In another possible implementation, a connection between the connecting member and the therapeutic device is a mortise-and-tenon joint, a housing of the wireless sensor and the connecting member are of an integrated structure, the connecting member being provided on the housing and including a corresponding interface for forming a concave-convex mating connection with the therapeutic device.

In another possible implementation, the corresponding interface is a protrusion or a recess provided on a peripheral surface of the housing, or, the corresponding interface is an internal thread, an external thread, or a spring provided at one end of the housing.

The implant provided in the present disclosure utilizes connecting member not only to connect the wireless sensor to the therapeutic device, but also to ensure the signals of the wireless sensor remain unaffected by the connecting member, enabling more precise signal transmission. Depending on the structural features of different therapeutic devices, various embodiments of the present disclosure present diverse structural configurations of connecting member. This approach not only prevents signal interference but also allows for the formation of various connection configurations between different types of therapeutic devices and wireless sensors according to the requirements of each application scenario, thereby achieving both therapeutic and diagnostic functions post-implantation.

In a second aspect, the present disclosure provides an implant for placement at a target site within a living body, including a stent, a wireless sensor and a connecting member. The stent is configured for being placed at the target site to enhance a physiological function of the living body; The therapeutic device is configured to be placed at the target site to enhance the physiological functions of the living body. The stent comprises a body formed by a plurality of elastic curved segments and at least one engagement segment, the engagement segment being disposed at a predetermined region of the body and connected to a portion of at least one of the elastic curved segments; the wireless sensor includes a monitoring element and a wireless signal transmission element; the monitoring element is configured to acquire target physiological parameters, and the wireless signal transmission element is configured to transmit the target physiological parameters acquired by the monitoring element to the exterior of the living body; wherein the connecting member is configured to connect the wireless sensor to the therapeutic device, the connecting member comprises a first connecting portion and a second connecting portion that are interconnected, the first connecting portion is connected to the wireless sensor, and the second connecting portion is connected to the engagement segment, thereby enabling the wireless sensor to be attached to the stent at the predetermined region.

In another possible implementation, the second connecting portion includes a clamped portion, and the engagement segment is inserted into the second connecting portion to clamp the clamped portion; and, the engagement segment is integrally formed with the portion of at least one elastic curved segment, and the engagement segment forces the clamped portion to abut against the peripheral surface of the body via its own elastic force, and/or, the clamped portion abuts against the peripheral surface of the body by the engagement segment with an adhesive.

In another possible implementation, the stent and the wireless sensor are arranged side by side, and the connecting member attaches the wireless sensor to the peripheral surface of the stent in the predetermined region.

In another possible implementation, the second connecting portion includes a sheet portion including an edge portion that forms the clamped portion, and the engagement segment is connected to the sheet portion by clamping the edge portion.

In another possible implementation, the edge portion is located at an edge region of the sheet portion or at an edge region of an opening formed on the sheet portion.

In another possible implementation, the connecting member is an annular sleeve, a part of the annular sleeve serves as the first connecting portion, and another part of the annular sleeve serves as the second connecting portion; an opening is provided on the annular sleeve, the edge portion of the sheet portion is formed by an edge portion of the annular sleeve, or, the edge portion of the sheet portion is formed by an edge portion of the opening.

In another possible implementation, the annular sleeve at least partially overlaps with the wireless signal transmission element in an axial direction; the annular sleeve is a non-metallic sleeve, and the cross-section of the non-metallic sleeve is an enclosed structure or an unclosed structure, or, the annular sleeve is the metallic sleeve, and a cross-section of the metallic sleeve is an unclosed structure.

In another possible implementation, the annular sleeve completely overlaps the wireless sensor in an axial direction, with the monitoring element being exposed on the end face of the wireless sensor, the monitoring element being configured to monitor pressure.

In another possible implementation, the elastic curved segment forms the body by extending circumferentially and axially, and the elastic curved segment includes at least a wave segment, a portion of the wave segment forms the engagement segment.

In another possible implementation, a predetermined trough portion and/or a predetermined crest portion of the wavy segment forms the engagement segment.

In another possible implementation, the engagement segment includes a first engagement segment and a second engagement segment, the second connecting portion includes a clamped portion, and the first engagement segment and the second engagement segment respectively clamp the clamped portion from two opposite directions.

In another possible implementation, the elastic curved segment forms the body by extending circumferentially and axially, and the elastic curved segment at least comprises a wave segment, a portion of which forms the first engagement segment and the second engagement segment. The first engagement segment and the second engagement segment include bent and turned ends, and the bent and turned ends of the first engagement segment and the second engagement segment are nested with each other.

In another possible implementation, the connecting member is an annular sleeve, wherein one portion of the annular sleeve serves as the first connecting portion, and another portion of the annular sleeve serves as the second connecting portion. The annular sleeve is provided with a first opening and a second opening spaced apart axially by a predetermined distance, and the first engagement segment and the second engagement segment are inserted through the first opening and the second opening, respectively, between the annular sleeve and the wireless sensor.

In another possible implementation, the stent is an atrial shunt device, the body is a tubular body for shunting, and the tubular body is formed by the elastic curved segments extending in a circular and/or helical pattern.

In another possible implementation, the tubular structure is configured to be radially resiliently deformable while maintaining a constant perimeter.

In another possible implementation, the tubular body is configured to be resiliently compressible along a first radial direction within a confined space, and/or the tubular body is configured to have a C-shaped cross-sectional profile when compressed along the first radial direction within the confined space.

In another possible implementation, the wireless sensor is arranged parallel to the body, and/or the wireless sensor is connected to the body tangentially and externally.

In another possible implementation, the stent further includes a clamping arm group, which comprises at least a first clamping arm and a second clamping arm. The first clamping arm and the second clamping arm being axially spaced apart along the body and distributed around the periphery of the body, with the first clamping arm and the second clamping arm each forming a cantilever protruding from the periphery of the body.

In another possible implementation, the first clamping arm and/or the second clamping arm is formed by bending an elastic metallic wire, and/or the first clamping arm and/or the second clamping arm is provided with a U-shaped or V-shaped structure formed by extending the elastic metallic wire radially for a predetermined distance and then turning back.

In another possible implementation, an elastic wire constituting the first clamping arm and/or the second clamping arm and an elastic wire of the tubular body to which the base of the first clamping arm and/or the second clamping arm is attached are the same elastic wire, and/or the first clamping arm and/or the second clamping arm have a predetermined distance from the respective adjacent end of the tubular body.

In another possible implementation, a free end of the first clamping arm overlaps with a free end of the second clamping arm in an axial projection, and a fixed end of the first clamping arm is staggered with a fixed end of the second clamping arm in the axial projection.

In another possible implementation, the stent further comprises a covering membrane, the covering membrane is provided outside or inside the body, and when the covering membrane is provided outside the body, the covering membrane is provided with a gap exposing the engagement segment.

In another possible implementation, the connecting member is made of polymer material and connects the wireless sensor to the stent via a heat-shrink process.

In a third aspect, the present disclosure provides an implant system, including a delivery catheter and an implant according the second aspect, the implant is compressed when being disposed within the delivery catheter, the stent being compressed into a C-shaped cross-sectional structure, an outer surface of the compressed stent at least partially surrounds the wireless sensor, and the implant being capable of being out from the catheter.

### BRIEF DESCRIPTION OF THE DRAWINGS

To provide a clearer explanation of the technical solutions in the embodiments of the present disclosure, the drawings referred for describing the embodiments will be briefly introduced below.

It should be understood that the following drawings only illustrate some embodiments of the present disclosure and should not be construed as limiting the scope.

It should also be understood that like or similar reference numerals are used in the drawings to designate like or similar elements.

It should also be understood that the drawings are merely schematic, the dimensions and/or the proportions of elements in the drawings may not be precise.
FIG. 1 is a schematic diagram of an implant in its application scenario according to an embodiment of the present disclosure.
FIG. 2 is a structural schematic diagram of the wireless sensor in the implant in FIG. 1.
FIG. 3 is a structural schematic diagram of the wireless sensor in the implant according to another embodiment.
FIG. 4 is a structural schematic diagram of the wireless sensor in FIG. 3 from another perspective.
FIG. 5 is a structural schematic diagram of the alternative connecting member in the implant in FIG. 3 according to another embodiment.
FIG. 6 is a schematic structural diagram of the housing of the wireless sensor in FIG. 3.
FIG. 7 is a structural schematic diagram of the wireless sensor in the implant according to another embodiment.
FIG. 8 is a schematic structural diagram of the housing of the wireless sensor in FIG. 7.
FIG. 9 is a schematic diagram of an implant in its application scenario according to another embodiment of the present disclosure.
FIG. 10 is a partial schematic cross-sectional view of the wireless sensor in FIG. 9.
FIG. 11 is a partial schematic cross-sectional view of the wireless sensor in an implant according to another embodiment of the present disclosure.
FIG. 12 is a schematic diagram of an implant in its application scenario according to another embodiment of the present disclosure.
FIG. 13 is an enlarged view of the implant in FIG. 12.
FIG. 14 is a structural schematic diagram of the wireless sensor in the implant in FIG. 12.
FIG. 15 is a structural schematic diagram of the wireless sensor in an implant according to another embodiment of the present disclosure.
FIG. 16 is a structural schematic diagram of the wireless sensor in an implant according to another embodiment of the present disclosure.
FIG. 17 is a structural schematic diagram of the wireless sensor in an implant according to another embodiment of the present disclosure.
FIG. 18 is a structural schematic diagram of the wireless sensor in an implant according to another embodiment of the present disclosure.
FIG. 19 is a schematic diagram showing the connection between the wireless sensor and part of the therapeutic device according to another embodiment of the present disclosure.
FIG. 20 is a structural schematic diagram of the wireless sensor in FIG. 19.
FIG. 21 is a structural schematic diagram of the wireless sensor in an implant according to another embodiment of the present disclosure.
FIG. 22 is a structural schematic diagram of the wireless sensor in an implant according to another embodiment of the present disclosure.
FIG. 23 is a structural schematic diagram of the wireless sensor in an implant according to another embodiment of the present disclosure.
FIG. 24 is a structural schematic diagram of the wireless sensor in an implant according to another embodiment of the present disclosure.
FIG. 25 is a structural schematic diagram of the housing of the wireless sensor according to another embodiment of the present disclosure.
FIG. 26 is a schematic diagram showing the connection between the wireless sensor including the housing in FIG. 25 and the therapeutic device.
FIG. 27 is a structural schematic diagram of the housing of the wireless sensor according to another embodiment of the present disclosure.
FIG. 28 is a schematic diagram showing the connection between the wireless sensor including the housing in FIG. 27 and the therapeutic device.
FIG. 29 is a schematic diagram showing the connection between the wireless sensor including the housing in FIG. 27 and the therapeutic device.
FIG. 30 is a schematic diagram showing the connection between the wireless sensor including the housing in FIG. 27 and the therapeutic device.
FIG. 31 is a schematic diagram of an implant in its application scenario according to another embodiment of the present disclosure.
FIG. 32 is a structural schematic diagram of the implant in FIG. 31.
FIG. 33 is a schematic diagram of an implant in its application scenario according to another embodiment of the present disclosure.
FIG. 34 is a structural schematic diagram of the implant in FIG. 33.
FIG. 35 is a schematic diagram of an implant in its application scenario according to another embodiment of the present disclosure.
FIG. 36 is a structural schematic diagram of the implant in FIG. 35.
FIG. 37 is a schematic diagram of an implant in its application scenario according to another embodiment of the present disclosure.
FIG. 38 is a structural schematic diagram of the implant in FIG. 37.
FIG. 39 is a schematic diagram of an implant in its application scenario according to another embodiment of the present disclosure.
FIG. 40 is a structural schematic diagram of a target site within a living body according to an embodiment of the present disclosure.
FIG. 41 is a structural schematic diagram of an implant according to an embodiment of the present disclosure.
FIG. 42 is a structural schematic diagram of the implant in FIG. 41, with the wireless sensor omitted.
FIG. 43 is a partially enlarged view showing the connection between the connecting member and the stent in FIG. 41.
FIG. 44 is a front view of the end face of the implant in FIG. 40.
FIG. 45 is a schematic diagram showing the compressed state of an implant within the delivery catheter according to an embodiment of the present disclosure.
FIG. 46 is a cross-sectional view of the delivery catheter and the implant in FIG. 45.
FIG. 47 is a schematic diagram showing the delivery catheter containing the implant as it arrives at the target site.
FIG. 48 is a schematic diagram showing the implant extending its one end and some clamping arms from the delivery catheter .
FIG. 49 is a schematic diagram showing the implant, wherein the delivery catheter is withdraw and the clamping arms of the implant arrives at the surface of the interatrial septum.
FIG. 50 is a schematic diagram showing the implant, wherein the delivery catheter is withdraw and the implant is placed at the target site.
FIG. 51 is a structural schematic diagram of the implant according to another embodiment of the present disclosure.
FIG. 52 is another structural schematic diagram of the implant in FIG. 51.
FIG. 53 is an enlarged view showing the connection between the connecting member and the engagement segment of the body in FIG. 51.
FIG. 54 is a structural schematic diagram of the body and the covering membrane of the implant in FIG. 51.
FIG. 55 is a structural schematic diagram of the wireless sensor, the connecting member and the engagement segment in the implant according to another embodiment of the present disclosure.
FIG. 56 is a structural schematic diagram of the wireless sensor, the connecting member and the engagement segment in the implant according to another embodiment of the present disclosure.
FIG. 57 is a structural schematic diagram of the wireless sensor, the connecting member and the engagement segment in the implant according to another embodiment of the present disclosure.
FIG. 58 is a structural schematic diagram of the wireless sensor, the connecting member and the engagement segment in the implant according to another embodiment of the present disclosure.
FIG. 59 is a structural schematic diagram of the wireless sensor, the connecting member and the engagement segment in one possible implement.
FIG. 60 is a schematic diagram of the connecting member in one possible implement.
FIG. 61a is front view of the profile shape of the stent in one possible implement.
FIG. 61b is front view of the profile shape of the stent in another possible implement.
FIG. 61c is front view of the profile shape of the stent in another possible implement.
FIG. 61d is front view of the profile shape of the stent in another possible implement.
FIG. 61e is front view of the profile shape of the stent in another possible implement.

### DETAILED DESCRIPTION OF THE EMBODIMENTS

The embodiments of the present disclosure are exemplarily described below in conjunction with the accompanying drawings. It should be understood that the implementation of the present disclosure may take various forms and should not be construed as limited to the embodiments set forth herein. The embodiments described herein are merely intended to provide a more thorough and complete understanding of the present disclosure.

It should be understood that the term 'comprising/including' and its variations used in the present disclosure are openended, meaning 'comprising/including but not limited to'. The term 'according to' means 'at least partially according to'.

It should be understood that although terms such as 'first' or 'second' may be used in the present disclosure to describe various elements, these elements are not limited by these terms, which are only used to distinguish one element from another.

The present disclosure provides an implant for placement at a target site within a living body, comprising a therapeutic device, a wireless sensor and a connecting member. The therapeutic device is configured to be placed at the target site to enhance a physiological function of the living body; the wireless sensor, for monitoring target physiological parameters within the living body, includes a monitoring element and a wireless signal transmission element; the monitoring element is configured to acquire target physiological parameters, and the wireless signal transmission element is configured to transmit the target physiological parameters acquired by the monitoring element to the exterior of the living body, for instance, to a remote controller; the connecting member connects a housing of the wireless sensor to the therapeutic device in a manner that preserves a signal transmission path of the wireless signal transmission element. Specifically, the connecting member preserves the signal transmission path of the wireless signal transmission element through the selection of one or more of its material, structure, and placement position, thus preventing the connecting member from obstructing or shielding the signal transmission path of the wireless signal transmission element. Of course, it is also possible for the operator to send wireless signals from a remote controller to the wireless signal transmission element, so as to achieve a bidirectional signal transmission, which is not limited in the present disclosure; the 'signal transmission path' referred to in the present disclosure can also be understood as a virtual path through which signals are transmitted from the wireless signal transmission element within the housing to the exterior of the living body, or through which signals are transmitted from the exterior of the living body to the wireless signal transmission element within the housing. When wireless signals are transmitted via the signal transmission path, the wireless signal transmission element has the capability of signal transmission.

Furthermore, the wireless sensor also includes a housing, the monitoring element and the wireless signal transmission element are disposed within the housing.

When the connecting member is made of metallic material, the connecting member partially encloses the housing (e.g., the connecting member is provided with an opening) to prevent the wireless signal transmission element of the wireless sensor from being obstructed or shielded by the connecting member. Alternatively, the connecting member and the wireless signal transmission element are arranged in a staggered manner to ensure the signals are not obstructed or shielded by the connecting member. When the connecting member completely encloses the housing, a non-electromagnetic shielding structure (such as a sleeve being made of non-metallic material) can be connected to the housing, ensuring the signal transmission path of the wireless signal transmission element is not obstructed or shielded by the connecting member. The housing, being made of non-metallic material like glass, does not block or shield the signal transmission path, either. Electromagnetic shielding structures herein refer to enclosed metal covers, metal cages, or similar structures that are capable of blocking electromagnetic signals. Non-electromagnetic shielding structures herein do not include those structures mentioned above and thus do not block electromagnetic signals. This allows the implant to simultaneously achieve therapeutic and diagnostic functions while ensuring the signals of the wireless sensors remain unaffected by the implant or the connecting member, enabling more precise signal transmission.

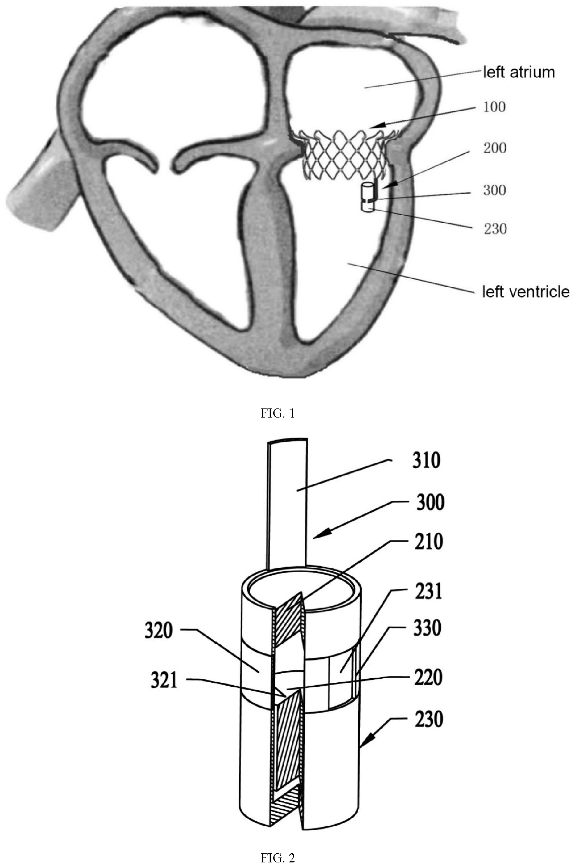

Refer to FIGS. 1 and 2. FIG. 1 is a schematic diagram of an implant in its application scenario according to an embodiment of the present disclosure. FIG. 2 is a structural schematic diagram of the wireless sensor 200 in the implant in FIG. 1. The implant includes a therapeutic device 100, a wireless sensor 200 and a connecting member 300. The wireless sensor 200 can be connected to the therapeutic device 100. In this embodiment, the therapeutic device 100 is a valve stent, which can be used to treat valve diseases occurring between the left atrium and left ventricle. The wireless sensor 200 is capable of monitoring fluid pressure and wirelessly transmitting related signals to an external receiver. In some possible implementations, the monitoring element may also be a device for measuring physiological parameters such as blood oxygen saturation, acceleration, pH, blood viscosity or temperature.

The therapeutic device 100 is a mesh-tubular stent including an expanded section and a non-expanded section, where the diameter of the expanded section is greater than that of the non-expanded section. In this embodiment, the wireless sensor 200 is connected to the lower edge of the non-expanded section of the therapeutic device 100.

The wireless sensor 200 includes a monitoring element 210 and a wireless signal transmission element 220. The monitoring element 210 is configured to acquire fluid pressure data. The wireless signal transmission element 220 is configured to wirelessly transmit the fluid pressure data to the exterior of the living body. In some possible implementations, the wireless sensor 200 further comprises a housing 230 accommodating the monitoring element 210 and the wireless signal transmission element 220. The housing 230 is a tubular structure, and a mating structure 231 is provided on a peripheral surface of the housing 230. The mating structure 231 is an annular groove connecting with the connecting member 300. In some possible implementations, the wireless sensor 200 may also include a power supply element for providing electrical energy to the monitoring element 210 and the wireless signal transmission element 220.

The connecting member 300 includes a hanging rod 310 and a clamping ring 320. The clamping ring 320 is an unclosed ring structure (i.e., a ring-shaped structure with an opening) and is provided with an opening 330 and an accommodation cavity 321. The handing rod 310 is fixed to the peripheral surface of the clamping ring 320 and extends in a direction parallel to an axial direction of the clamping ring 320. The connecting member 300 is sleeved onto the mating structure 231 of the housing 230 via the clamping ring 320. The connecting member 300 is secured below the therapeutic device 100 via the handing rod 310, thereby connecting the wireless sensor 200 to the therapeutic device 100. Specifically, one end of the handing rod 310 can be welded to the mesh of the therapeutic device 100, or can be connected to the therapeutic device 100 by crimping, bonding, etc., or it can be integrally formed with at least a portion of the therapeutic device 100. In some possible embodiments, the handing rod 310 may also be replaced by other connecting structures, such as anchoring hooks, sleeves, insertion rods, etc. The connecting structures can be selected based on the categories of therapeutic devices.

In this embodiment, the connecting member 300 is made of metallic material, thus ensuring a robust connection between the therapeutic device 100 and the wireless sensor 200. The clamping ring 320 of the connecting member 300 is an unclosed sleeve with thin walls, which is not circumferentially enclosed (a longitudinal opening is provided on the wall), thus avoiding electromagnetic signal shielding and the significant increasing on its radial dimensions when connected to the housing 230. Additionally, the housing 230 is provided with a corresponding mating structure 231 (an annular groove) to accommodate the clamping ring 320, ensuring the peripheral surface of the wireless sensor 200 remains smooth without protrusions or grooves, thereby preventing thrombus formation. Moreover, the connection between the clamping ring and the annular groove provides an axial positioning, enhancing the connection strength therebetween. In other possible implementations, there is no annular groove provided on the peripheral surface of the housing 230, and the entire or partial peripheral surface serves as the mating structure. The clamping ring 320 is directly sleeved onto the entire or partial peripheral surface of the housing 230. Further, the interface between the clamping ring 320 and the housing 230 can be reinforced by adhesion therebetween to enhance connection strength. In other possible implementations, the housing 230 and the connecting member 300 may be integrally formed, thus reducing size, increasing strength while simplifying the assembly process. Additionally, the clamping ring 320 is provided with an opening 330, ensuring no shielding of signals from the wireless signal transmission element 220. In other possible implementations, the clamping ring 320 is an unclosed ring being made of metallic material, and an axial length of the clamping ring 320 cooperates with that of the housing 230. An opening provided on the clamping ring may have an axial length equal to or less than that of the housing 230, but greater than that of the wireless signal transmission element 220, and the opening is aligned to the wireless signal transmission element 220 of the wireless sensor 200, thereby forming a wireless signal transmission window for signals transmission.

In some possible embodiments, the clamping ring 320 is an enclosed ring structure (i.e., a ring-shaped structure without an opening). Correspondingly, to avoid obstructing the transmission of wireless signals, the wireless signal transmission element 220 and the monitoring element 210 are distributed at opposite ends of the lumen, and the axial position of the mating structure 231 of the housing 230 is misaligned to the axial position of the wireless signal transmission element 220. That is, the connecting member 300 clearance effectively preserves the signal transmission path of the wireless signal transmission element 220. In other possible embodiments, the clamping ring 320 is an enclosed ring that completely overlaps the housing 230 axially but is made of non-shielding material, such as polymer material, rendering it a non-shielding structure that does not obstruct the wireless signals of the wireless signal transmission element 220, thereby preserving the signal transmission path. The signal transmission path may be a predetermined region, extending in a specific direction from a certain position of the wireless signal transmission element 220 (e.g., a cylindrical region corresponding to the peripheral surface of the wireless signal transmission element 220). The signal transmission path may also be a region facing the entire or partial the peripheral surface of the wireless signal transmission element 220. The present disclosure is not intended to limit here.

In some possible embodiments, the number of hanging rods 310 may be two or more, which enhances the connection strength between the connecting member 300 and the therapeutic device 100. In some possible embodiments, the connecting member 300 may further include an upper cover or a lower cover. The upper cover or the lower cover is connected to the clamping ring 320 and is sleeved onto one end of the housing 230. Preferably, the upper cover or lower cover is integrally formed with the clamping ring 320. In some possible embodiments, the therapeutic device 100 includes a mesh stent formed by cutting a metallic tube, the hanging rod 310, the clamping ring 320 and the mesh stent are integrally formed by cutting. Alternatively, the hanging rods 310, clamping ring 320, the upper cover or the lower cover and the mesh stent are integrally formed by cutting, then an expansion process is applied to it to form the mesh stent with the expanded section. This method further simplifies the connection process between the therapeutic device 100 and the housing 230, increasing the connection strength therebetween.

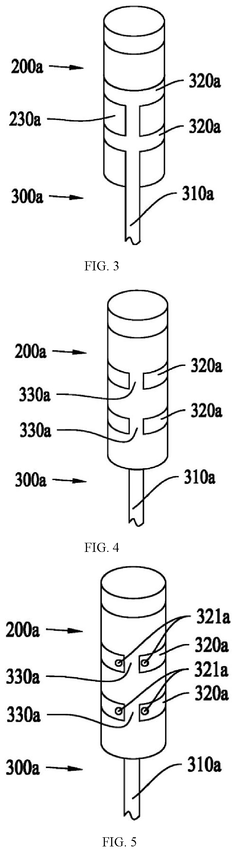

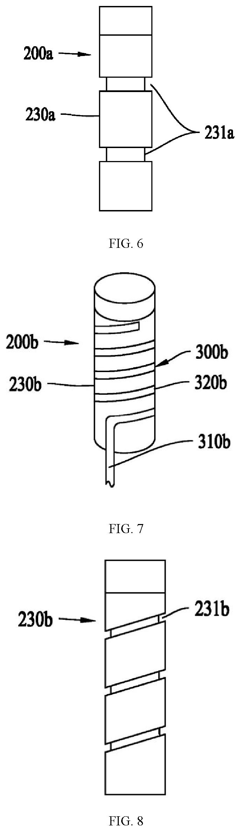

Refer to FIGS 3 to 5. FIG. 3 is a structural schematic diagram of the wireless sensor in the implant according to another embodiment. FIG. 4 is a structural schematic diagram of the wireless sensor in FIG. 3 from another perspective. FIG. 5 is a structural schematic diagram of the alternative connecting member in the implant in FIG. 3 according to another embodiment. FIG. 6 is a schematic structural diagram of the housing of the wireless sensor in FIG. 3.

In this embodiment, the connecting member 300a is made of metallic material. The connecting member 300a includes a hanging rod 310a and two clamping rings 320a. Each clamping ring 320a is provided with an opening 330a. The two clamping rings 320a are spaced apart axially by a predetermined distance. A portion of the hanging rod 310a connects the two clamping rings 320a in series. The wireless sensor 200a is a tubular body, including two mating structures 231a provided on the surface of its housing 230a. In this embodiment, the mating structures 231a are specifically annular grooves, and the axial distance between the two annular grooves is equal to the axial distance between the two clamping rings 320a. The two clamping rings 320a of the connecting member 300a are respectively embedded in the two mating structures 231a of the housing 230a. Since there are two engagement, disposed axially, between the connecting member 300a and the housing 230a, the connection therebetween is more robust. The opening 330a on each clamping ring 320a makes the clamping ring 320a an unclosed ring, thereby avoiding interference with wireless signals. In the implementation shown in FIG. 5, reinforcement holes 321a are further provided, near the opening 330a, on each clamping ring 320a. By threading a reinforcement member, such as a suture, through the two reinforcement holes 321a, the connection strength between the connecting member 300a and the housing 230a can be further enhanced. In some possible implementations, the peripheral surface of the housing 230a may not have annular grooves, and the two clamping rings 320a may directly sleeved onto the peripheral surface of the housing 230a.

The aforementioned wireless sensor 200a can be connected to the therapeutic device 100 via the hanging rod 310a. The therapeutic device 100 may be the valve stent shown in FIG. 1.

Refer to FIGS. 7 and 8. FIG. 7 is a structural schematic diagram of the wireless sensor in the implant according to another embodiment. FIG. 8 is a schematic structural diagram of the housing 230b of the wireless sensor 200b in FIG. 7. In this embodiment, the portion of the connecting member 300b being connected to the housing 230b of the wireless sensor 200b is a helical structure 320b, while the portion being connected to the therapeutic device 100 is a handing rod 310b. The handing rod 310b and the helical structure 320b are formed by bending and coiling the same metallic strip. The surface of the housing 230b is provided with a mating structure 231b, specifically a helical groove. The connecting member 300b is embedded into the helical groove of the housing 230b via the helical structure 320b, ensuring a tight connection therebetween. Moreover, the helical structure 320b is formed by helically winding a metallic strip, without forming any electromagnetic shielding structure (e.g., a metallic enclosed ring structure), thereby avoiding to obstruct the transmission of wireless signals. The helical structure 320b forms an unclosed tubular structure.

The aforementioned wireless sensor 200b can be connected to a therapeutic device 100 via the hanging rod 310b. The therapeutic device 100 may be the valve stent shown in FIG. 1.

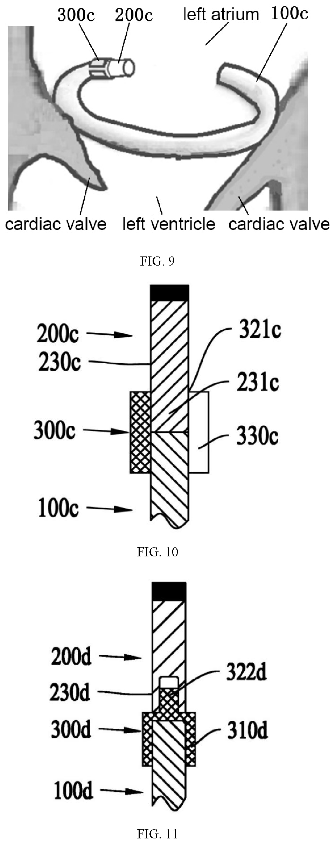

Refer to FIGS. 9 and 10. FIG. 9 is a schematic diagram of an implant in its application scenario according to another embodiment of the present disclosure. FIG. 10 is a structural schematic diagram of the wireless sensor in FIG. 9. In this embodiment, the implant includes a therapeutic device 100c and a wireless sensor 200c. The therapeutic device 100c is an unclosed annuloplasty ring. The annuloplasty ring shown in FIG. 9 is placed between two valves, the two valves are located between the left atrium and left ventricle of the heart. Annuloplasty ring primarily treats symptoms caused by annular pathologies or valvular insufficiency resulting from other cardiac diseases, restoring native valvular functions through annular correction.

The wireless sensor 200c is connected to one end of the annuloplasty ring via a connecting member 300c. The connecting member 300c is an unclosed sleeve, that is, a sleeve including an opening 330c extending axially along the wall of the sleeve. The sleeve includes an accommodating cavity 321c. One end of the sleeve is sleeved onto a terminal segment 231c of the wireless sensor 200c, while another end of the sleeve is sleeved onto one terminal end of the therapeutic device 100c.

Specifically, the connecting member 300c can be made of either metallic or polymer material. An inner diameter of the accommodating cavity 321c of the connecting member 300c may be slightly lower than an outer diameter of the housing 230c and the therapeutic device 100c, allowing for a tight connection through interference fitting. Alternatively, the connecting member 300c could be a heat-shrink tube, which forms a tight connection between the housing 230c and the therapeutic device 100c via heat-shrink process. Furthermore, the connection between the connecting member 300c and both the therapeutic device 100c and the housing 230c can be reinforced with adhesive bonding. If the connecting member 300c is made of metallic material, the opening is arranged to be aligned to the wireless signal transmission element disposed within the wireless sensor 200c. In some possible embodiments, the connecting member 300c may also be a complete sleeve without any openings, enclosing the terminal segment 231c of the housing 230c. The connecting member 300c can be made of either metallic or polymer material. If the connecting member 300c is made of metallic material, the wireless signal transmission element in the wireless sensor 200c is not located at the terminal segment 231c-that is, the wireless signal transmission element is placed away from the connecting member 300c-thus ensuring the connecting member 300c does not obstruct wireless signal transmission.

Referring to FIG. 11, FIG. 11 is a structural schematic cross-sectional view of the wireless sensor in an implant according to another embodiment of the present disclosure. In this embodiment, the therapeutic device 100d is an annuloplasty ring. The wireless sensor 200d is connected to one terminal end of the annuloplasty ring via a connecting member 300d. One terminal end of a housing 230d of the wireless sensor 200d is provided with a connection hole. The connecting member 300d includes a sleeve portion 310d and an insertion portion 322d, which are coaxially arranged. The sleeve portion 310d is sleeved onto one terminal end of the therapeutic device 100d. The insertion portion 322d is inserted into the connection hole at the terminal end of the housing 230d. The insertion portion 322d is secured to the connection hole by adhesive, threaded structures or welding. In some possible implementations, the connecting member 300d consists solely of the insertion portion 322d. One end of the insertion portion 322d is inserted into the housing 230d and another end is inserted into the therapeutic device.

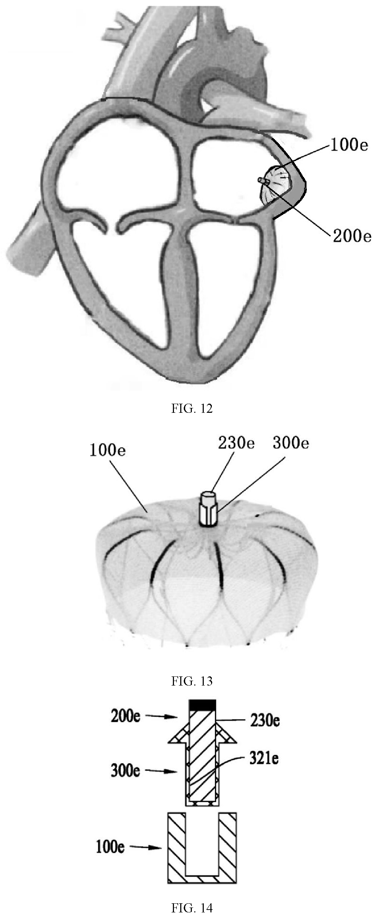

Refer to FIGS. 12 to 14. FIG. 12 is a structural schematic diagram of an implant in its application scenario according to another embodiment of the present disclosure. FIG. 13 is an enlarged view of the implant in FIG. 12. FIG. 14 is an assembly schematic diagram of the wireless sensor and the therapeutic device in the implant in FIG. 12. In this embodiment, the therapeutic device 100e is a left atrial appendage occluder. The connecting member 300e is a sleeve with one end enclosed, and the accommodating cavity 321e of the sleeve accommodates the housing 230e of the wireless sensor 200e. The left atrial appendage occluder is generally discoid, including a cup-shaped connection structure at its center. The housing 230e of the wireless sensor 200e and the connecting member 300e are inserted into the cup-shaped connection structure of the left atrial appendage occluder. The connecting member 300e may be formed by adhesive curing, which firmly connects the therapeutic device 100e to the housing 230e of the wireless sensor. The wireless signal transmission element in the wireless sensor 200e is offset from the cup-shaped connection structure of the therapeutic device 100e. In some possible implementations, the connecting member 300e may also include the structure shown in FIG. 10 or FIG. 11, while the center of the left atrial appendage occluder is provided with a rod-shaped connection structure, which could similarly connect the left atrial appendage occluder to the wireless sensor.

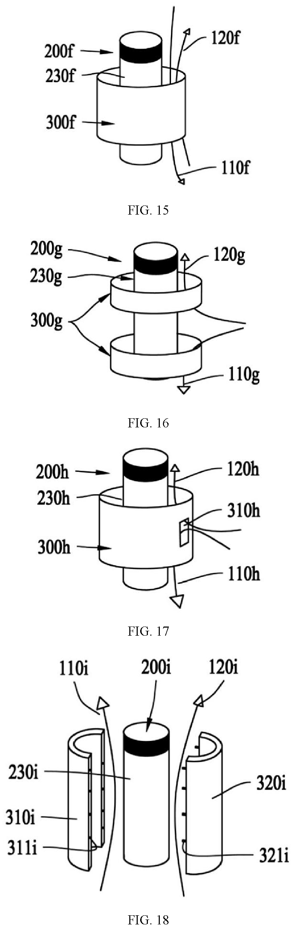

Referring to FIG. 15, FIG. 15 is a structural schematic diagram of the wireless sensor in an implant according to another embodiment of the present disclosure. In this embodiment, the wireless sensor 200f is a cylindrical structure, including a cylindrical housing 230f for accommodating the monitoring element and the wireless signal transmission element. The connecting member 300f is a polymer heat-shrinkable sleeve. The therapeutic device is a stent-type instrument, such as a vascular stent, a shunt, or an occluder. A first connecting wire 110f and a second connecting wire 120f can be extended from the stent-type instrument. The first connecting wire 110f and the second connecting wire 120f are inserted, between the housing 230f and the heat-shrinkable sleeve, respectively from two edges of the sleeve. After heating, the heat-shrinkable sleeve contracts, thus clamping the first connecting wire 110f and the second connecting wire 120f. The first connecting wire 110f and the second connecting wire 120f may be metallic wires with certain strength. This connection therebetween allows the wireless sensor 200f to be connected to the outer surface, inner surface, or end of the therapeutic device. In some possible embodiments, the connecting member 300f may also be a metallic sleeve with an inner diameter adapted to an outer diameter of the housing 230f. When the connecting member 300f is sleeved onto the housing 230f, the connecting member 300f can be axially offset from the wireless signal transmission element accommodate within the housing 230f, so as to prevent shielding of the wireless signals. In other embodiments, the connecting member 300f may also be an unclosed metallic sleeve, such as a sleeve with a longitudinal opening on its wall, axially aligned to the wireless signal transmission element within the housing 230f to prevent shielding of the wireless signals.

Referring to FIG. 16, FIG. 16 is a structural schematic diagram of the wireless sensor in an implant according to another embodiment of the present disclosure. In this embodiment, the housing 230g of the wireless sensor 200g is cylindrical, within which the monitoring element and the wireless signal transmission element are accommodated. The connecting member 300g includes two arranged sleeves. The therapeutic device is a stent-like instrument, such as a vascular stent, a shunt, or an occluder. Filamentous structures can be extended from the stent-like instrument to connect with the sleeves. In this embodiment, the filamentous structures are a first connecting wire 110g and a second connecting wire 120g, both connected to the connecting member 300g. Specifically, the first connecting wire 110g and the second connecting wire 120g are inserted, between the housing 230g and the sleeves, respectively from the one edge of one of the two sleeves. The first connecting wire 110g passes through the lower sleeve and extends from a middle section downwards, while the second connecting wire 120g passes through the upper sleeve and extends from the middle section upwards. When the connecting member 300g is a heat-shrinkable tube, its initial diameter is larger than that of the housing 230g. When the connecting member 300g is a metallic tube, it has an inner diameter adapted to the outer diameter of the housing 230g. In some possible embodiments, the filamentous structures may also be bent into U-shaped or V-shaped configurations. In other possible implementations, the filamentous structures may be bent into q-shaped or inverted q-shaped configurations, where the ends of the wires may loop after passing through the sleeve to prevent detachment. Furthermore, the ends of two filamentous structures are inserted from opposite directions and may be interlocked with each other, thereby further securing the connection. In some possible implementations, lamellar structures can be used to replace the connecting wires. The lamellar structures have smaller thickness but greater strength, thus increasing connection strength.

Referring to FIG. 17, FIG. 17 is a structural schematic diagram of the wireless sensor in an implant according to another embodiment of the present disclosure. In this embodiment, the wireless sensor 200h is substantially the same as the wireless sensor 200f in FIG. 15, with the difference being the presence of an opening 310h of the wireless sensor 200h. The first connecting wire 110h and the second connecting wire 120h are not inserted from the edges of the sleeve but from the opening 310h of the sleeve, respectively extending toward the axial two ends of the wireless sensor 200h. When the connecting member 300h is a heat-shrink tube, its initial diameter is larger than that of the housing 230h. When the connecting member 300h is a metallic tube, it has an inner diameter adapted to the outer diameter of the housing 230h.

Referring to FIG. 18, FIG. 18 is a structural schematic diagram of the wireless sensor in an implant according to another embodiment of the present disclosure. The housing 230i of the wireless sensor 200i is a tubular body. The connecting member is a sleeve formed by combining a first semi-shell 310i and a second semi-shell 320i, equivalent to two parts of a sleeve split longitudinally. A longitudinal end surface of the first semi-shell 310i is provided with a connecting hole 311i, and the corresponding longitudinal end surface of the second semi-shell 320i is provided with a positioning pin 321i. The first semi-shell 310i and the second semi-shell 320i are connected as a whole via the cooperation of the connecting hole 311i and the positioning pin 321i. The therapeutic device is a stent-like instrument, such as a vascular stent, a shunt or an occluder. The first connecting wire 110i and the second connecting wire 120i can be extended from the stent-like instrument and connected to the wireless sensor 230i. The first connecting wire 110i and the second connecting wire 120i are abut against the surface of the housing 230i respectively by the pressing of the first semi-shell 310i and the second semi-shell 320i.

It can be understood that in some embodiments shown in FIGS. 15 to 18, the first connecting wire and the second connecting wire can either be the metallic wires forming the body of the stent-like instrument or the filamentous structures formed by cutting a metallic tube, or other filamentous structures connected to the body of the stent-like instrument by processes such as welding or bonding. The first connecting wire and the second connecting wire can also be replaced by Lamellar structures. The first connecting wire and the second connecting wire may also be referred to as the first engagement segment and the second engagement segment, respectively. The body of the stent-like instrument may further include an elastic curved segment, which is connected to the first engagement segment and the second engagement segment.

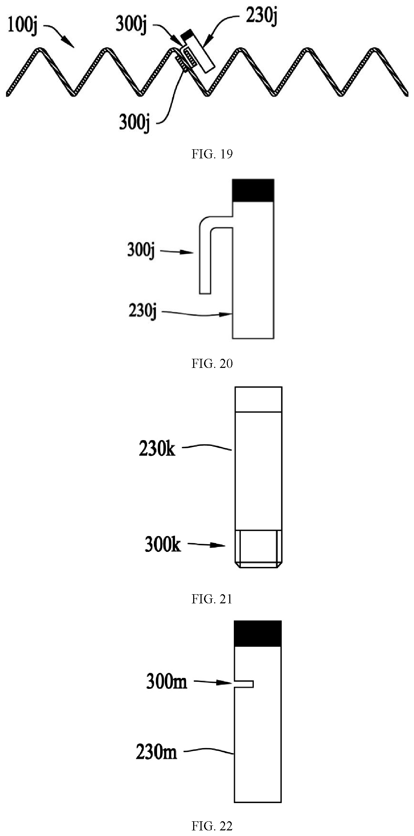

Refer to FIGS. 19 and 20. FIG. 19 is a schematic diagram showing the connection between the wireless sensor and part of the therapeutic device according to another embodiment of the present disclosure. FIG. 20 is a structural schematic diagram of the wireless sensor in FIG. 19. In this embodiment, the therapeutic device 100j is a stent-like instrument, such as a vascular stent, a valve stent, a shunt or an occluder. The stent-like instrument may be formed by braiding or winding elastic wires, or by cutting a tubular structure into a mesh stent. The housing 230j and the connecting member 300j may be integrally formed. The connecting member 300j includes an insertion rod and a sleeve provided on the surface of the housing 230j. The sleeve is sleeved onto the filamentous structures of the stent-like instrument, and the insertion rod is inserted between the filamentous structure and the sleeve. The sleeve can be made of metallic material or polymer material. The insertion rod includes a portion perpendicular to the housing 230j and a portion parallel to the housing 230j, the portion parallel to the housing 230j has a small spacing from the surface of the housing and is configured to be inserted into the sleeve.

Referring to FIG. 21, FIG. 21 is a structural schematic diagram of the wireless sensor in an implant according to another embodiment of the present disclosure. In this embodiment, the housing 230k and the connecting member 300k of the wireless sensor are integrally formed and made of non-metallic material. The housing 230k is a tubular body, within which the monitoring element and the wireless signal transmission element are accommodated, including an external thread structure provided at its one end for connecting with the therapeutic device. Corresponding thread hole may be provided on the therapeutic device. The therapeutic device may be an instrument such as an occluder or a leadless pacemaker.

Referring to FIG. 22, FIG. 22 is a structural schematic diagram of the wireless sensor in an implant according to another embodiment of the present disclosure. In this embodiment, the housing 230m of the wireless sensor is integrally formed with the connecting member 300m and is made of non-metallic material. The housing 230m is a tubular body, within which the monitoring element and the wireless signal transmission element are accommodated. The connecting member 300m is a groove provided on the peripheral surface of the housing 230m, configured to form a mortise-and-tenon joint with a corresponding protrusion provided on the therapeutic device. The mortise-and-tenon joint refers to a connection formed by the cooperation of concave and convex. The therapeutic device can be a stent-like instrument, such as a vascular stent, a valve stent, a shunt or an occluder, where grooves can directly engage with the wires of the stent. Alternatively, the therapeutic device may also be provided with a male connecting structure (such as a pin), which can be inserted into the groove of the housing 230m.

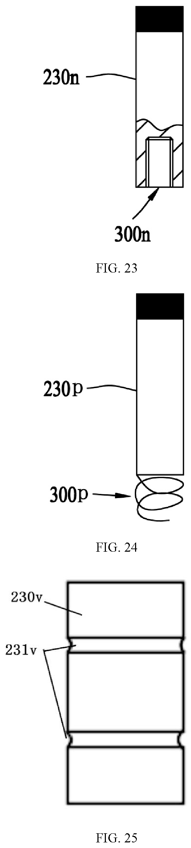

Referring to FIG. 23, FIG. 23 is a structural schematic diagram of the wireless sensor in an implant according to another embodiment of the present disclosure. In this embodiment, the housing 230n of the wireless sensor is integrally formed with the connecting member 300n and is made of non-metallic material. The housing 230n is a tubular body, within which the monitoring element and the wireless signal transmission element are accommodated, including a threaded hole provided on an end surface of its one end. The end with threaded hole serves as the connecting member 300n. The therapeutic device can be provided with corresponding external thread structures to connect with the connecting member 300n. Specifically, the therapeutic device may be an instrument such as an occluder or an annuloplasty ring. A threaded rod can be disposed at a center of the occluder or one end of the annuloplasty ring to secure the connecting member 300n.

Referring to FIG. 24, FIG. 24 is a structural schematic diagram of the wireless sensor in an implant according to another embodiment of the present disclosure. In this embodiment, the housing 230p is a tubular body, within which the monitoring element and the wireless signal transmission element are accommodated. The therapeutic device may be a leadless pacemaker, or the leadless pacemaker can be accommodated within the housing 230p, the housing 230p effectively serves as the connecting member. One end of the housing 230p is provided with a connecting spring 300p, coaxially disposed at its terminal end. The connecting spring can anchor at the affected site. In some possible embodiments, the connecting spring 300p serves as the connecting member, with no therapeutic device accommodated within the housing 230p, and the sensor connects to the therapeutic device via the connecting spring 300p.

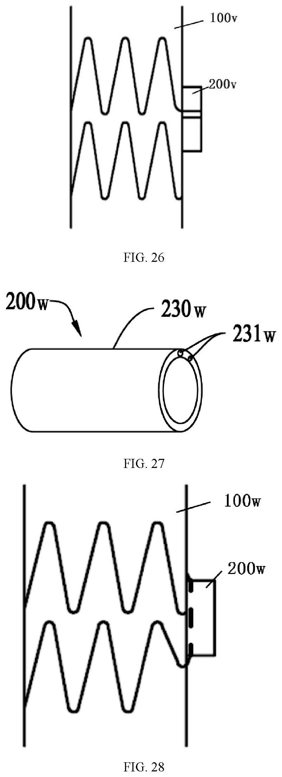

Refer to FIGS. 25 and 26. FIG. 25 is a structural schematic diagram of the housing of the wireless sensor according to another embodiment of the present disclosure. FIG. 26 is a schematic diagram showing the connection between the wireless sensor including the housing in FIG. 25 and the therapeutic device. In this embodiment, the housing 230v is integrally formed with the connecting member. Specifically, the connecting member consists of two annular grooves 231v located at the peripheral surface of the housing 230b. The therapeutic device is a stent-like instrument, such as a cardiac stent formed by braiding metallic wires. The stent-like instrument may incorporate two connecting wires that engaged in the two annular grooves 231v of the housing 230v, allowing the wireless sensor to be arranged side by side on the exterior or interior of the therapeutic device. The therapeutic device in FIG. 26 may be an interatrial shunt device.

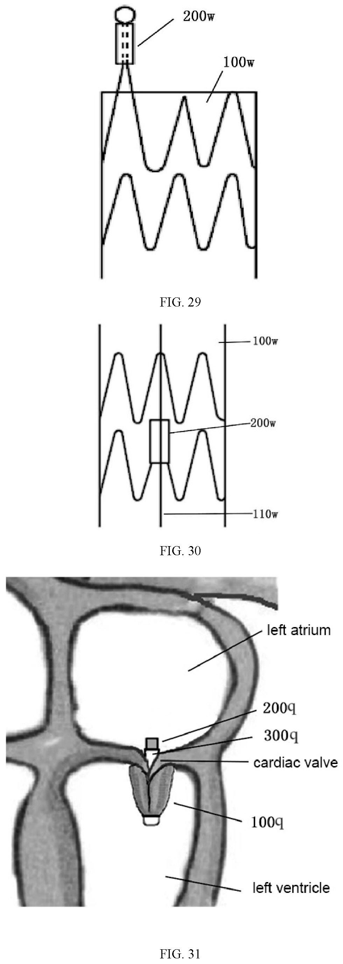

Refer to FIGS. 27 to 30. FIG. 27 is a structural schematic diagram of the housing of the wireless sensor according to another embodiment of the present disclosure. FIG. 28 is a schematic diagram showing the connection between the wireless sensor including the housing in FIG. 27 and the therapeutic device. FIG. 29 is a schematic diagram showing the connection between the wireless sensor including the housing in FIG. 27 and the therapeutic device. FIG. 30 is a schematic diagram showing the connection between the wireless sensor including the housing in FIG. 27 and the therapeutic device.

In this embodiment, the connecting member of the wireless sensor 200w is integrally formed with the housing 230w and is made of a non-metallic material. The housing 230w is a cylindrical shell, within which the monitoring element and the wireless signal transmission element are accommodated. The connecting member consists of at least one through-hole 231w, parallel to an axial direction, provided on a wall of the housing 230w. Preferably, the connecting member includes two through-holes 231w, parallel to the axial direction, provided on a wall of the housing 230w. The therapeutic device is a stent-type instrument, such as a vascular stent, an atrial septal shunt stent, or an aneurysm/dissection isolation stent formed by braiding wires. In the implementation shown in FIG. 28, two connecting wires are extended from the stent-type instrument and passed through the through-holes 231w of the housing 230w, enabling the wireless sensor 200w and the therapeutic device to be closely abut with each other, side by side. In the implementation shown in FIG. 29, one connecting wire is extended from the stent-type instrument. The wire passes through one through-hole 231w, bends into a loop with a larger diameter, then passes back through another through-hole 231w, and continues bending to form a part of the stent. The wireless sensor 200w can be placed at terminal ends, internal center, internal surface, or outer surface of the stent as needed, ensuring it is securely threaded into a body of the stent-type instrument. In the implementation shown in FIG. 30, the therapeutic device 100w is further provided with a reinforcement rib 110w. The reinforcement rib 110w passes through one of the through-holes 231w of the housing 230w to fasten the wireless sensor 200w onto the therapeutic device 100w.

It should be understood that in the embodiments shown in FIGS. 27 to 30, the connecting wire can either be the metallic wire forming the body of the stent-like instrument or a filamentary structure formed by cutting a metallic tube. Alternatively, it may be another filamentary structure connected to the body of the stent-like instrument through processes such as welding or bonding. The connecting wire may also be referred to as a engagement segment. The body of the stent-like instrument may further include an elastic curved segment connected to the engagement segment.

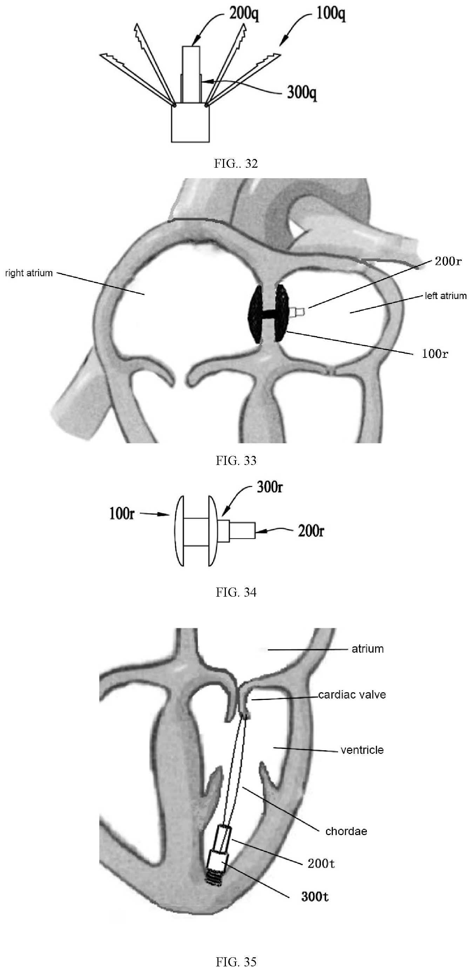

Refer to FIGS. 31 and 32. FIG. 31 is a schematic diagram of an implant in its application scenario according to another embodiment of the present disclosure. FIG. 32 is a structural schematic diagram of the implant in FIG. 31. In this embodiment, the therapeutic device 100q is a valve clip. The valve clip includes two pairs of clips and a body portion. The two pairs of clips are arranged on one side of the body portion, and the wireless sensor 200q is inserted into the body portion of the valve clip via a connecting member 300q. Specifically, the connecting member 300q is a sleeve, with one end of the sleeve sleeved onto the wireless sensor 200q and another end inserted into the body portion of the valve clip, the another end locates on the same side as the clips. In some possible implementations, the wireless sensor 200q may also be inserted into another side of the body portion of the valve clip, with the wireless sensor 200q and the clips located on opposite sides of the body portion. Alternatively, one end of the wireless sensor 200q may be located on one side of the body portion of the valve clip, while another end of the wireless sensor 200q is located on the opposite side of the body portion, enabling simultaneous monitoring of the atrium and ventricle.

Refer to FIGS. 33 and 34. FIG. 33 is a schematic diagram of an implant in its application scenario according to another embodiment of the present disclosure. FIG. 34 is a structural schematic diagram of the implant in FIG. 33. In this embodiment, the therapeutic device 100r of the implant is an atrial septal defect occluder. The therapeutic device 100r includes two anchoring discs and a connecting portion therebetween. The wireless sensor 200r is inserted into a middle section of the connecting portion. Specifically, the connecting member 300r is a sleeve, one end of the sleeve sleeved onto the end of the wireless sensor 200r and another end inserted into the middle section of the connecting portion of the therapeutic device 100r.

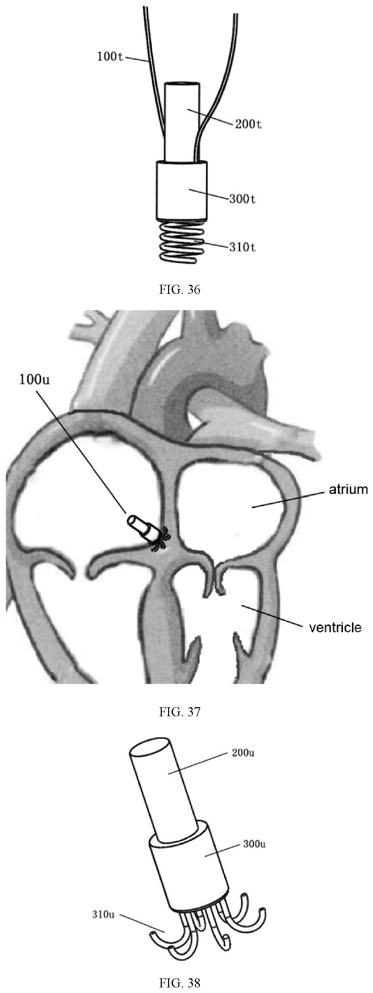

Refer to FIGS. 35 and 36. FIG. 35 is a schematic diagram of an implant in its application scenario according to another embodiment of the present disclosure. FIG. 36 is a structural schematic diagram of the implant in FIG. 35. In this embodiment, the therapeutic device 100t is a chordae tendineae repair element, including an artificial chordae and an anchoring member 310t. The connecting member 300t is a sleeve that abuts the artificial chordae against a surface of the wireless sensor 200t. The artificial chordae and the anchoring member 310t are distributed at both ends of the connecting member 300t. The anchoring member 310t is a spring anchored at the cardiac apex; the artificial chordae is fixed to the valve to pull the valve.

Refer to FIGS. 37 and 38. FIG. 37 is a schematic diagram of an implant in its application scenario according to another embodiment of the present disclosure. FIG. 38 is a structural schematic diagram of the implant in FIG. 37. In this embodiment, the therapeutic device 100u is a leadless pacemaker, enveloped by the connecting member 300u. The leadless pacemaker is coaxially fixed to the wireless sensor 200u via the connecting member 300u. The entire implant is secured within the heart by an anchor hook 310u of the leadless pacemaker.

It can be understood that when the connecting members 300r, 300t, and 300u are made of metallic material, they are either unclosed sleeves or axially offset from the wireless signal transmission elements of the wireless sensors 200r, 200t, and 200u.

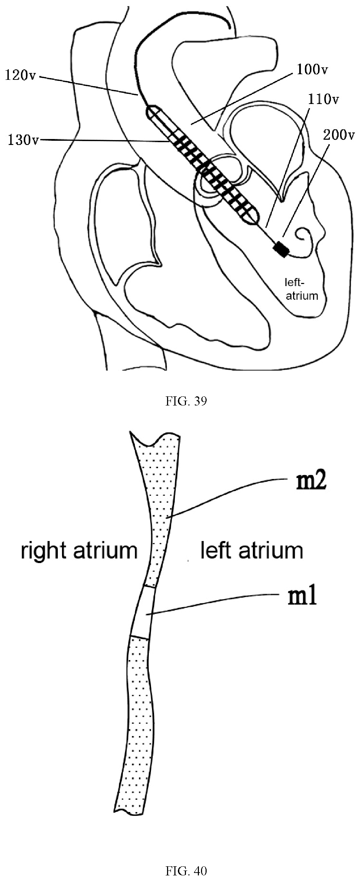

Referring to FIG. 39, FIG. 39 is a schematic diagram of an implant in its application scenario according to another embodiment of the present disclosure. In this embodiment, the therapeutic device 100v is a cardiac catheter pump assembly. The cardiac catheter pump assembly can be placed in the left ventricle to guide blood from the left ventricle to the ascending aorta. The cardiac catheter pump assembly is a short-term implant, including a guide wire 110v, a catheter 120v, and a pump body 130v. The guide wire 110v and the catheter 120v will also remain in the living body with the pump body 130v for a period of time. In this embodiment, the wireless sensor 200v can be placed on the guide wire 110v or the catheter 120v. In this case, the connecting member can adopt the structure shown in FIG. 27, where the guide wire 110v passes through the through-hole 231w of the housing 230w, or as shown in FIG. 19, where a sleeve is sleeved onto the catheter 120v, and an insertion rod is inserted between the catheter 120v and the sleeve. When the wireless sensor 200v is located on the catheter 120v, its position in the heart is the ascending aorta. When the wireless sensor 200v is located on the guide wire 110v, its position in the heart is the left ventricle. The wireless sensor 200v can also be placed on the outer surface of the pump body 130v. Since the pump body 130v has a mesh-like structure similar to a stent, the connecting member can adopt any of the forms shown in FIGS.. 15 to 20 or FIGS.. 25 to 27. In this case, its position in the heart is the left ventricle.

Hereafter, to better understand the present disclosure, an implant and an implant system are described using the example of their application in cardiovascular scenarios.

Atrial septostomy is a tool to alleviate left atrial hypertension by creating an opening in the interatrial septum to divert blood flow from the left atrium to the right atrium, thereby reducing the load on the left atrium. Atrial septostomy can be categorized into implant-based and non-implant-based approaches. Implant-based septostomy requires securing the implant within the artificially created interatrial septal opening to prevent its closure. Currently, interatrial septal perforation and implant fixation are generally performed through vascular interventional techniques.

In addition to the aforementioned shunt devices suitable for heart failure patients, there are other stent-like implants designed for placement within the heart or blood vessels for patients with cardiovascular diseases. For instance, some patients may require occluders to optimize cardiovascular functions, while those with vascular stenosis might need vascular stents. However, beyond treating symptoms with implants, monitoring hemodynamic parameters of cardiovascular systems of patients is also highly significant. For heart failure patients, for example, atrial pressure monitoring serves as a crucial health management tool alongside surgical interventions. Nevertheless, integrating pressure monitoring devices with these stent-like implants presents a major technical challenge in the field. The applicant has observed that when pressure monitoring devices are mounted within stents-which are typically mesh structures woven from metallic wires or cut from tubes-the mesh can interfere with or even block the wireless signal transmission of the monitoring device. Alternatively, placing the pressure monitoring device axially at the proximal or distal end of the stent increases the overall structural length, potentially obstructing normal blood flow, affecting stent port dimensions, and raising the risk of long-term stenosis.

The embodiments of the present disclosure provide an implant configured to be placed within a living body to enhance physiological functions while acquiring target physiological parameters. The implant comprises a stent, a wireless sensor and a connecting member. The stent is intended for placement at a target site to enhance the physiological functions of the living body and can be a mesh structure such as a shunt, an occluder or vascular stent. The stent includes at least a body formed by multiple elastic curved segments. Herein, the term 'elastic curved segment' refers to a wire segment that is elastic and generally curved, though it may exhibit straight sections locally. These segments can be formed by a single wire or by multiple different wires, such as woven metallic filaments, without limitation in the present disclosure. The wireless sensor includes a monitoring element or a wireless signal transmission element being electrically connected with. The monitoring element is used to acquire target physiological parameters, while the wireless signal transmission element transmits these parameters to the exterior of the living body. The connecting member connects the wireless sensor to the stent, positioning the sensor outside the stent and adjacent to a peripheral surface of the stent. This arrangement ensures that the stent does not interfere with signal transmission from the wireless sensor, nor does the sensor affect the dimensions of port at either end of the stent-particularly when the stent is a shunt, preserving its shunt capability.

The stent and wireless sensor may be secured through various methods such as crimping, bonding, suturing, or welding. In this embodiment, they are fixed via a specially designed connecting member, which includes a first connecting portion and a second connecting portion interconnecting with each other. The first connecting portion connects to the wireless sensor, while the second connecting portion connects to elastic curved segments of the stent. Specifically, the stent further includes at least one engagement segment located in a predetermined region of the body and connected to a portion of at least one elastic curved segment. The engagement segment is inserted into the second connecting portion, allowing the connecting member to adhere to the stent in the predetermined region (e.g., the surface of the stent, preferably its peripheral surface). The wireless sensor is thereby adjacent to outer periphery of the stent in a roughly side-by-side configuration. By utilizing the engagement segment connected to the elastic curved segments to secure the wireless sensor to the surface of the stent, the connection structure is simple, reliable, and compact. Since the elastic curved segments are inherently part of the stent, no additional structures are needed to interface with the connecting member, avoiding significant increases in the radial dimension of the implant and further reducing the risk of thrombus formation.

FIG. 40 is a structural schematic diagram of a target site within a living body according to an embodiment of the present disclosure. Specifically, the target site ml is a hole in an interatrial septum m2 located between the left atrium and the right atrium. The hole may be an unclosed foramen ovale or an artificially created aperture. For patients with heart failure, such hole can reduce left atrial pressure and alleviate heart failure symptoms. The stent provided in this embodiment of the disclosure serves as a shunt, which can be placed within the hole to maintain its shape and guide blood flow from the left atrium to the right atrium. Preferably, the wireless sensor is placed at the middle section of the peripheral surface of the shunt. Thus, when the implant in this embodiment is placed in the interatrial septum m2, the wireless sensor and the stent are placed together within the target site ml and in contact with it. Furthermore, two monitoring elements can be placed at both ends of the wireless sensor to simultaneously measure pressures in the left atrium and the right atrium. It should be noted that the wireless sensor can also be placed at other suitable locations on a shunt depending on specific application requirements, as long as signal transmission remains unaffected.

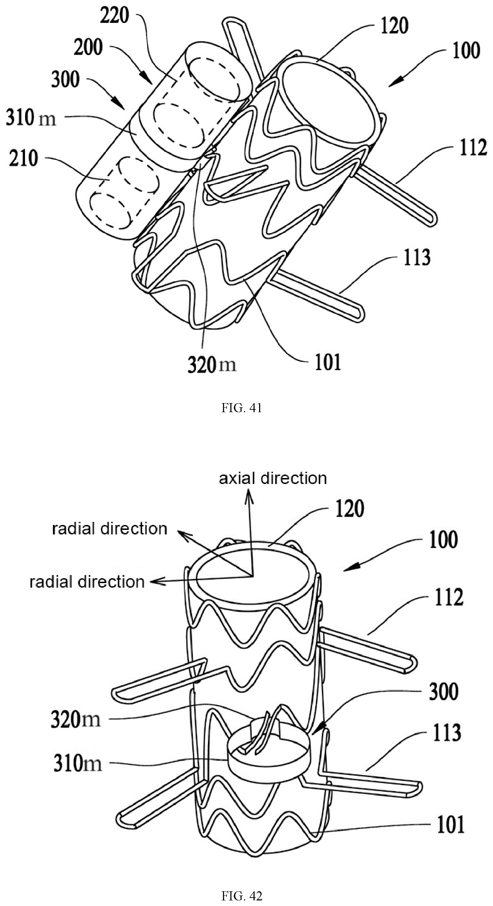

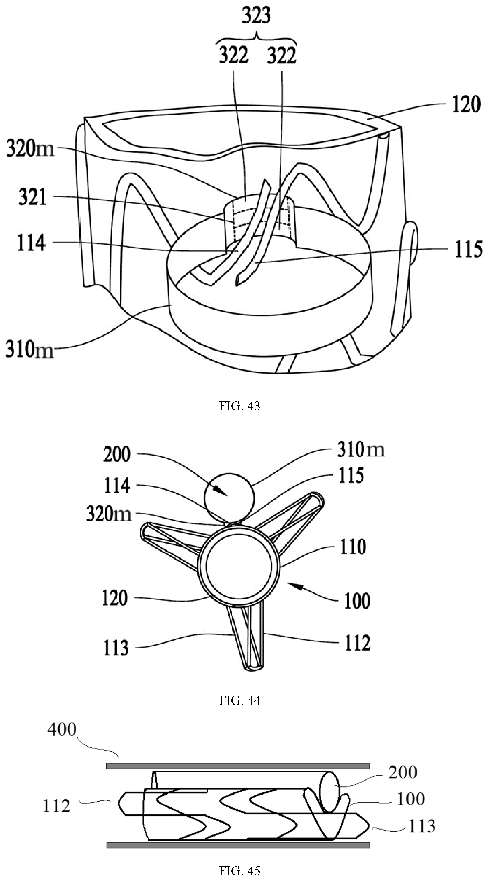

Refer to FIGS. 41 to 44. FIGS. 41 to 44 are structural schematic diagrams of an implant according to an embodiment of the present disclosure. In this embodiment, the implant includes a stent 100 and a wireless sensor 200. FIG. 41 is a structural schematic diagram of an implant according to an embodiment of the present disclosure. FIG. 42 is a structural schematic diagram of the implant in FIG. 41, with the wireless sensor omitted. FIG. 43 is a partially enlarged view showing the connection between the connecting member and the stent in FIG. 41. FIG. 44 is a front view of the end face of the implant in FIG. 40.

The implant includes a stent 100, a wireless sensor 200 and a connecting member 300. The stent 100 and the wireless sensor 200 are arranged side by side, and the connecting member 300 connects them, forming as a whole. It should be understood that the term 'arranged side by side' in the present disclosure is not limited to parallel placement; the stent 100 and the wireless sensor may also form a predetermined angle (but not perpendicular to each other). Preferably, the stent 100 and the wireless sensor 200 are arranged in parallel. As shown in FIG. 44, when the body of the stent is a tubular structure and the wireless sensor 200 is also a tubular structure, under the action of the engagement segment and the connecting member, the wireless sensor 200 and the stent 100 exhibit an externally tangent configuration, which allows the implant to achieve a smaller radial dimension during delivery, facilitating crimping within the delivery device.

In this embodiment, the stent 100 includes a body 110, a first clamping arm 112, a second clamping arm 113 and a covering membrane 120. The body 110 is a tubular structure formed by braiding metallic wires, and is generally cylindrical. The covering membrane 120 is applied to the inner and/or outer surface of the body 110. The first clamping arm 112 and the second clamping arm 113 extend radially outward from the surface of the body 110 to form a cantilever structure. The first clamping arm 112 and the second clamping arm 113 form at least one clamping arm group, distributed on either side of the atrial septum m2 to clamp it. The body 110, the first clamping arm 112 and the second clamping arm 113 are all formed by winding and bending elastic curved segments 101. Specifically, the body 110 is divided into an upper tubular segment and a lower tubular segment. There are three first clamping arms 112 and three second clamping arms 113. The three first clamping arms 112 are uniformly distributed on the surface of the upper tubular segment, while the three second clamping arms 113 are uniformly distributed on the surface of the lower tubular segment. Some elastic curved segments 101 extend in a wavy pattern axially, while extend circumferentially to form a mesh-tubular body; another portion of the elastic curved segments 101 extends radially outward from the surface of the mesh-tubular body and then turns back to the surface of the mesh-tubular body, forming U-shaped clamping arms.

In some possible embodiments, the body 110 may also be formed by cutting metals. The number of first clamping arms 112 and second clamping arms 113 may each be greater or fewer than three, such as two, as long as the clamping function is achieved. The elastic curved segments 101 may also take other configurations, which are not limited in the present disclosure.

Furthermore, the three first clamping arms 112 and three second clamping arms 113 form three clamping arm groups. The first clamping arm 112 and the second clamping arm 113 of each clamping arm group are axially spaced apart. The first clamping arm 112 has a predetermined spacing from the first end (upper end in FIG. 41) of the body 110. The second clamping arm 113 has a predetermined spacing from the second end (lower end in FIG. 41) of the body 110. The free ends of the first clamping arm 112 and the second clamping arm 113 overlap in axial projection, while the fixed ends of the first clamping arm 112 and the second clamping arm 113 are offset in axial projection, thereby achieving a larger clamping area (the area of the clamping region formed jointly by the first clamping arm 112 and the second clamping arm 113) and obtaining better clamping effects. Herein, the fixed ends of the first clamping arm 112 and the second clamping arm 113 refer to the ends where they are connected to the body 110. The upper tubular segment and the lower tubular segment are coaxially distributed and may be connected via a covering membrane 120 and/or an elastic curved segment. The elastic curved segment of the lower tubular segment initially extends 360° circumferentially in an annular form and then helicals upward. The elastic curved segment of the upper tubular segment initially extends upward in a helical form circumferentially and completes the final circle in an annular form. In the above implementation, the axial distance of the clamping arms is 3mm. The optional range for the axial distance is greater than 0mm and less than or equal to 3mm. In the above implementations, the elastic metallic wire constituting the clamping arm and the elastic metallic wire of the mesh-tublar body to which the base of the clamping arm is connected are the same elastic metallic wire. In some possible implementations, the clamping arm may be a separate U-shaped or V-shaped component welded to the elastic curved segment of the mesh-tubular body.

The configuration of clamping arms in this embodiment differs from that in the prior art presenting an anchoring with a waist-shaped or a flange-structured. It effectively reduces the volume of the shunt within the cavity and the contact area of the shunt with anchoring points, avoiding adverse effects on blood flow, while securely anchoring the body at the target site.

In this embodiment, the length of the covering membrane 120 equals the sum of the lengths of the upper and lower tubular segments. As the covering membrane 120 is a tubular structure with certain strength, when disposed on the inner/outer surface of the body 110, it can enhance the strength of the body 110 and the connection between the upper and lower tubular segments, while maintaining the predetermined morphology of the elastic curved section, thereby preserving the shape of the body 110. The covering membrane 120 ensures a smooth inner lumen of the tube, preventing tissue hyperplasia from obstructing the tubular cavity and maintaining its patency. The material of the covering membrane 120 can be a polymer or biological material, including but not limited to polyester, polytetrafluoroethylene, or animal pericardium. A coating may be applied to the surface of the covering membrane 120 to improve surface smoothness, thereby enhancing long-term patency rates. In some possible embodiments, the covering membrane 120 can also be placed on the outer surface of the body 110. In other possible implementations, both the inner and outer surfaces of the body 110 may be covered with a layer of the covering membrane 120. Another beneficial effect of the covering membrane 120 is its insulating function, which can to some extent isolate the body 110 from the wireless sensor 200, thus preventing signal interference. The connection between the covering membrane 120 and the body 110 can be achieved through heat pressing or suturing. For clarity in distinguishing the covering membrane 120 from the body 110 in the diagram, the thickness of the covering membrane 120 has been intentionally exaggerated and shown on the inner surface of the body 110, whereas its actual thickness is smaller than shown.

Those skilled in the art will understood that although in this embodiment, the aforementioned stent 100 is connected to the wireless sensor 200 via a connecting member 300, in other embodiments, the aforementioned stent 100 may also be used as an independent shunt.

In this embodiment, the engagement segment includes a first engagement segment and a second engagement segment, each integrally formed with a respective portion of the two elastic curved segments. In other words, the first engagement segment is naturally extended from a portion of one elastic curved segment, while the second engagement segment is naturally extended from a portion of the other elastic curved segment. Referring to FIGS. 42 and 43, at the middle section of the body 110, the initial segment (an upper portion) of the elastic curved segment of the lower tubular segment forms the first engagement segment 114, and the terminal segment (a lower portion) of the elastic curved segment of the upper tubular segment forms the second engagement segment 115.

As shown in FIG. 41, the wireless sensor 200 includes a monitoring element 210 and a wireless signal transmission element 220. The monitoring element 210 may be a pressure monitoring element capable of measuring, for example, blood flow pressure. The monitoring element 210 is placed at one end of the wireless sensor 200 for monitoring left atrial pressure. The wireless signal transmission element 220 transmits signals acquired by the monitoring element 210 to the exterior of the living body via electromagnetic waves. The monitoring element 210 and the wireless signal transmission element 220 are encapsulated as a single unit by a sealed structure (also referred to as a housing). In other embodiments, two monitoring elements 210 may also be placed at both ends of the wireless sensor 200 to measure both left and right atrial pressures, with the wireless signal transmission element 220 placed between the two monitoring elements 210.

Refer to FIGS. 42 and 43. In this embodiment, the connecting member 300 is an annular sleeve, with a partial segment of the annular sleeve slightly protruding outward to form a second connecting portion 320m, while the remaining parts constitute a first connecting portion 310m. The outward protrusion of the second connecting portion 320m may be formed by the tension between the stent 100 and the wireless sensor 200 when connected by the connecting member 300, or it may be a permanent protrusion applied during manufacturing. The first connecting portion 310m is wrapped around the middle section of the wireless sensor 200. The second connecting portion 320m is overall a lamellar structure with thin walls, including a sheet portion 321. The sheet portion 321 is roughly rectangular in shape. It has two parallel edges, namely an upper edge and a lower edge perpendicular to the axis of the annular sleeve. The upper edge and at least part of its adjacent area form one edge portion 322, while the lower edge and at least part of its adjacent area form another edge portion 322. These two edge portions 322 are designed to be clamped by the first engagement segment 114 and the second engagement segment 115, meaning the two edge portions 322 constitute the clamped portion 323.

In this embodiment, the width of the annular sleeve (dimension in the axial direction) is relatively small, while the lengths of the first engagement segment 114 and the second engagement segment 115 are relatively large. Therefore, the first engagement segment 114 and the second engagement segment 115 each clamps two edge portions 322, meaning the first engagement segment 114 and the second engagement segment 115 clamp the entire axial region of the sheet portion 321. In some possible embodiments, the axial dimension of the sheet portion 321 is larger, and/or the lengths of the first engagement segment 114 and/or the second engagement segment 115 are smaller. In such cases, the first engagement segment 114 may only clamp the edge portion 322 corresponding to the lower edge, while the second engagement segment 115 may only clamp the edge portion 322 corresponding to the upper edge. Relative to the overall size of the connecting member, the protrusion height of the second connecting portion 320m is very small. In some possible embodiments, the first connecting portion may also be a complete tubular body, and the second connecting portion 320m can be considered as being arranged along the outer profile of the first connecting portion 310m, ensuring that the radial dimensions of the second connecting portion 320m and the first connecting portion 310m do not significantly increase. The term 'fitting' mentioned here can mean that two adjacent surfaces are in contact with each other (but can be separated by external forces, such as being pried apart by the insertion of an engagement segment), or that the two adjacent surfaces are very close to each other, allowing the first engagement segment to be inserted.

Through the above-mentioned connection methods, the second connecting portion 320m of the connecting member 300 is clamped between the first engagement segment 114, the second engagement segment 115, and the covering membrane 120, effectively pressing the second connecting portion 320m against the surface of the body 110. The stent100 and the wireless sensor 200 are connected in parallel and externally tangent to each other. Additionally, the first engagement segment 114 and the second engagement segment 115 are also clamped between the wireless sensor 200 and the second connecting portion 320m. This connection is simple and reliable, facilitating the assembly and connection of small elements like shunts. Moreover, the free ends of the first engagement segment 114 and the second engagement segment 115 point in opposite directions, further preventing the second connecting portion 320m from detaching in the axial direction. The connection method, where the engagement segments on the surface of the stent100 clamp the lamellar structure on the surface of the wireless sensor 200, results in a very small radial dimension for the connection structure. This allows the wireless sensor 200 and the stent100 to be tightly connected without causing the overall radial dimension of the implant to become excessively large.

The shape of the connecting member 300 is not limited to the structure described in the previous embodiment, as long as the first connecting portion 310m can securely connect to the wireless sensor 200, and the second connecting portion 320m is arranged to conform to the surface of the first connecting portion 310m and can be clamped by the engagement segments of the body 110. Therefore, the first connecting portion 310m can also be a ring-shaped structure with an opening, which can encircle the wireless sensor 200; or an enclosed or unclosed box-like structure adapted to the shape of the wireless sensor 200, capable of accommodating it; or a structure designed to match the connected portion of the wireless sensor 200, such as featuring snap-fit, threaded, or adhesive surfaces (of course, the wireless sensor itself must have corresponding mating structures like slots, threads, or adhesive surfaces). The second connecting portion 320m, meanwhile, can be an arched or cantilevered structure that can be clamped by the engagement segments.

Preferably, the connecting member 300 is a sleeve, with one part being the first connecting portion and the other part being the second connecting portion. When the connecting member 300 and the wireless signal transmission element 220 at least partially overlap axially, the connecting member 300 is a non-metallic sleeve, and the cross-section of the non-metallic sleeve is an enclosed structure (e.g., an O-shaped structure) or an unclosed structure (e.g., an unclosed structure), so as to avoid the connecting member 300 itself affecting signal transmission; or the ring-shaped sleeve is a metallic sleeve, and the cross-section of the metal sleeve is an unclosed structure, which can also avoid the connecting member 300 itself affecting signal transmission; when the connecting member 300 and the wireless signal transmission element 220 do not overlap axially at all, there is no restriction on the material of the connecting member 300.

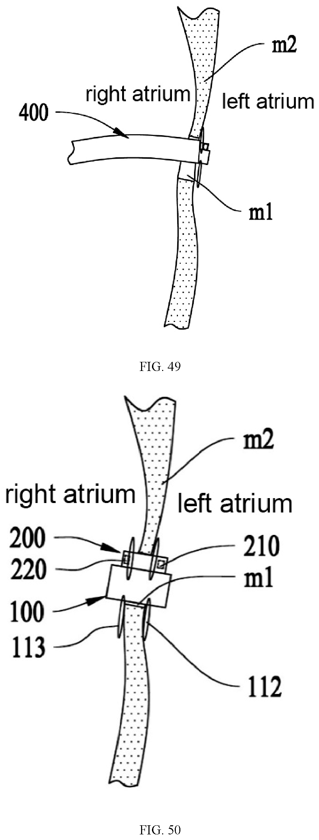

Referring to FIG. 50, which shows the implant wherein the implant is placed at the target site. The target site ml is a hole connecting the left atrium and the right atrium. The entire implant is inserted into the hole, with the first clamping arm 112 and the second clamping arm 113 grasping the atrial septal tissue to prevent the implant from axially dislodging from the target site ml. The monitoring element 210 is located in the left atrium and can measure the blood pressure there. When the left atrium contracts, some blood flows into the left ventricle, while another portion passes through the lumen of the stent 100 into the right atrium. The wireless sensor 200 is arranged side by side outside the stent 100, ensuring that it does not obstruct blood flow within or at either end of the lumen of the stent. Moreover, since the stent 100 does not enclose the wireless sensor 200, it does not block wireless signal transmission. The wireless sensor 200 can be implanted side by side with the stent 100 in the atrial septal hole, avoiding excessive occupation of atrial space as seen in traditional axial tandem arrangements. The connecting member 300 tightly connects with the engagement segment of the stent 100, ensuring that the stent 100 and the wireless sensor 200 are firmly pressed against each other. This not only enhances their connection strength but also reduces their radial dimensions, facilitating transvascular interventional implantation.

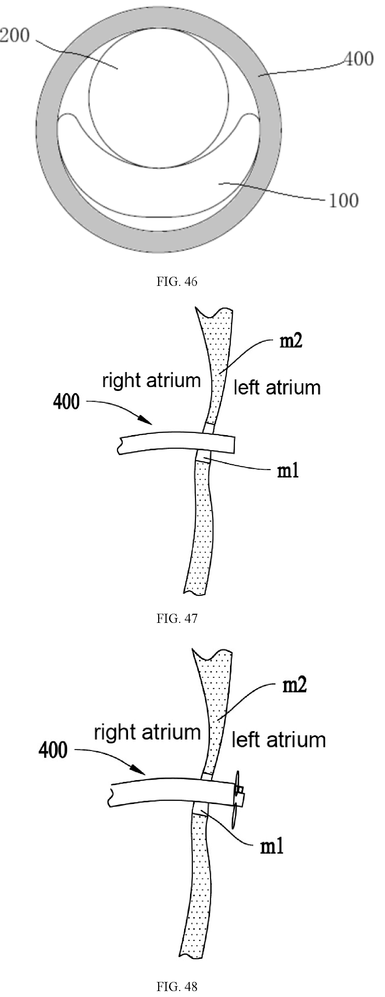

Refer to FIGS. 45 and 46. FIG. 46 is a schematic diagram showing the compressed state of the implant when placed within the delivery catheter. FIG. 47 is a cross-sectional schematic of the delivery catheter and implant in FIG. 46. The stent 100 of the implant is formed by elastic curved segments 101, allowing the entire structure to deform under external compression and return to its original shape upon removal of the force. The implant, together with the delivery catheter used for the interventional procedure, forms an implant system.

In the implant, the elastic curved segments constitute the body of the stent 100, endowing the entire stent 100 with elasticity. When pressure is applied to the wireless sensor 200 along the central connecting line between the wireless sensor 200 and the stent 100, the stent 100 can be compressed. That is, the stent 100 is resiliently deformable along the first radial direction within a confined space (the radial dimension decreases in the direction of the applied force while increasing perpendicular to it; here, the 'first radial direction' refers to a radial direction aligned with the force). The wireless sensor 200 can partially embed into the recessed area of the stent 100. Upon removal of the external force, the stent 100 will revert to its original shape and enhance its positional relationship with the wireless sensor 200. During the process of placing the implant into the delivery catheter 400, the catheter wall exerts pressure on both the stent 100 and the wireless sensor 200 along and perpendicular to the central connecting line, compressing the stent 100 along the first radial direction into a C-shaped cross-section, while the wireless sensor 200 partially embeds into the recessed portion of the stent 100. Additionally, the first clamping arm 112 and the second clamping arm 113 are flattened along the longitudinal section to adhere to the surface of the stent 100, meaning the free ends of the first clamping arm and the second clamping arm move closer to the extension directions of the ends of the stent 100.. Once placed in the delivery catheter 400, the overall radial dimensions of the implant are compressed. Since the compression deformation involves flattening the tubular body without altering its circumference-rather than reducing radial dimensions through axial elongation-the axial dimensions of the implant remain largely unchanged. A shorter implant length facilitates bending of the delivery catheter 400, enhancing procedural convenience.

Referring collectively to FIGS. 47 to 49, these illustrate the surgical process of implanting the shunt device into the target site via an interventional catheter. FIG. 47 is a schematic diagram showing the delivery catheter containing the implant as it arrives at the target site. FIG. 48 is a schematic diagram showing the implant with its one end and some clamping arms being out of the delivery catheter . FIG. 49 is a schematic diagram showing the implant, wherein the delivery catheter is withdraw and the clamping arms of the implant arrives at the surface of the interatrial septum. FIG. 50 is a schematic diagram showing the implant, wherein the delivery catheter is withdraw and the implant is placed at the target site.

One end of the delivery catheter 400 containing the implant enters the right atrium through a larger blood vessel in the human body and passes through the hole in the interatrial septum m2 into the left atrium. At this position, the implant is partially out of the distal end of the delivery catheter 400 by either withdrawing the catheter or pushing the implant outward, causing the first clamping arm 112 to deploy into a cantilevered state. After the first clamping arm 112 is deployed, the overall radial dimension of the implant exceeds the diameter of the hole in the interatrial septum m2. Further withdrawal of the delivery catheter moves the implant until the first clamping arm 112 contacts the interatrial septum m2. Continued withdrawal of the delivery catheter 400 causes the implant to remain in place due to the obstruction of the interatrial septum m2, without retracting further with the catheter. Once the implant is completely detached from the delivery catheter 400, the second clamping arm 113 also deploys, securing the stent 100 and the wireless sensor 200 within the hole of the interatrial septum m2 via the first clamping arms 112 and second clamping arms 113. The stent 100 then resumes its original shape in its radial direction, with its lumen allowing blood flow from the left atrium into the right atrium. After the stent 100 is in place, the monitoring element 210 of the wireless sensor 200 can monitor left atrial blood pressure in real time and transmit relevant data to an external receiving device via the wireless signal transmission element 220.

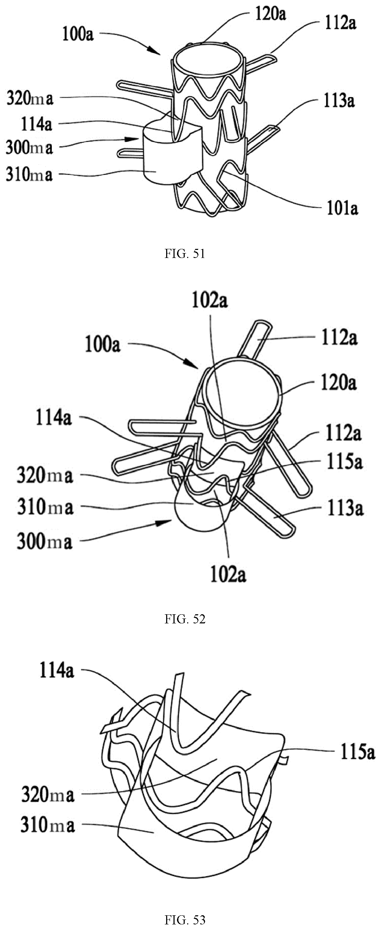

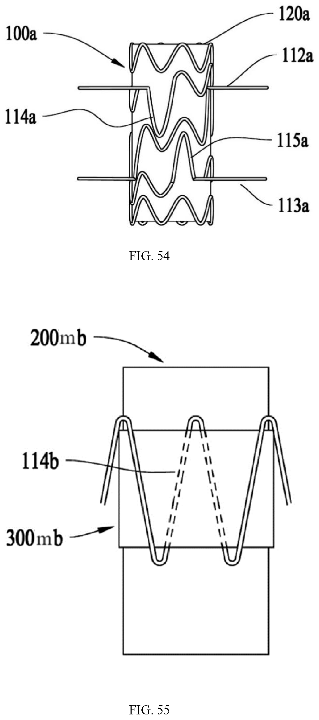

Refer to FIGS. 51 to 54. FIG. 51 is a structural schematic diagram of an implant according to another embodiment of the present disclosure. FIG. 52 is another structural schematic diagram of the implant in FIG. 51. FIG. 53 is an enlarged view showing the connection between the connecting member and the engagement segment of the body in FIG. 51. FIG. 54 is a structural schematic diagram of the body of the implant in FIG. 51. The implant in this embodiment is largely identical in structure to the implant in the previous embodiment, such as the first clamping arm 112a, the second clamping arm 113a, and the covering membrane being constructed similarly to the first clamping arm 112, the second clamping arm 113, and the covering membrane 120. The primary difference lies in the structure of the connecting member and the corresponding engagement segment on the body. For clarity in illustrating the structure of the connecting member, the sensor is omitted in the schematic diagram of this embodiment.

In this embodiment, the elastic curved segment 101a extends circumferentially and axially to form the body of the stent 100a, with the elastic curved segment being entirely wavy in shape and comprising multiple wave segments 102a. Among these, the trough portion 114a of one wave segment 102a in the middle of the stent 100a forms the first engagement segment, while the crest portion 115a of another wave segment 102a in the middle of the stent 100a forms the second engagement segment. The trough portion 114a and the crest portion 115a enclose a larger area both axially and circumferentially, providing greater strength and clamping force, thereby enabling a more tight connection.

The connecting member 300ma is an annular sleeve with open ends. In this embodiment, the width (axial dimension) of the annular sleeve is greater than that of the annular sleeve in the embodiment shown in FIG. 41. The annular sleeve comprises two parts: one part forms a first connecting portion 310ma with a cross-section resembling a semi-open two-thirds circular ring, while the other part forms a second connecting portion 320ma with a cross-section resembling a semi-open, roughly rectangular sleeve. Of course, in other implementations, a diaphragm may be added at the junction to divide the sleeve into two cavities. The first connecting portion 310ma surrounds the wireless sensor. The second connecting portion 320ma is designed for clamping by the first engagement segment 114a (trough portion) and the second engagement segment 115a (crest portion). The second connecting portion 320ma is essentially a rectangular thin film sheet pressed against the surface of the body 100a by the first engagement segment 114a (trough portion) and the second engagement segment 115a (crest portion). Additionally, the second connecting portion 320ma may be further bonded or sutured to the covering membrane 120a to enhance connection strength.

Referring to FIG. 55, FIG. 55 is a schematic structural diagram of the connecting member 300mb and the engagement segment 114b in an implant according to another embodiment of the present disclosure. To more clearly illustrate the connection relationship between the connecting member and the engagement segment, this embodiment only shows the sensor 200mb, the connecting member 300mb, and part of the elastic curved segment structure in the stent, omitting other structures. The connecting member 300mb is an annular sleeve formed from a polymer material. The upper edge, lower edge, and the region between them of one part of the annular sleeve form the clamped portion, which in this embodiment is the second connecting portion. The other part of the annular sleeve is the first connecting portion, which is fixed around the middle of the sensor 200mb. The engagement segment 114b in the stent is an inverted V-shaped structure formed by bending an elastic curved segment, which is inserted between the second connecting portion and the sensor 200mb from the lower edge of the second connecting portion. The length of the engagement segment 114b is greater than the axial dimension of the connecting member 300mb, so one end of the engagement segment 114b extends out from between the connecting member 300mb and the sensor 200mb.

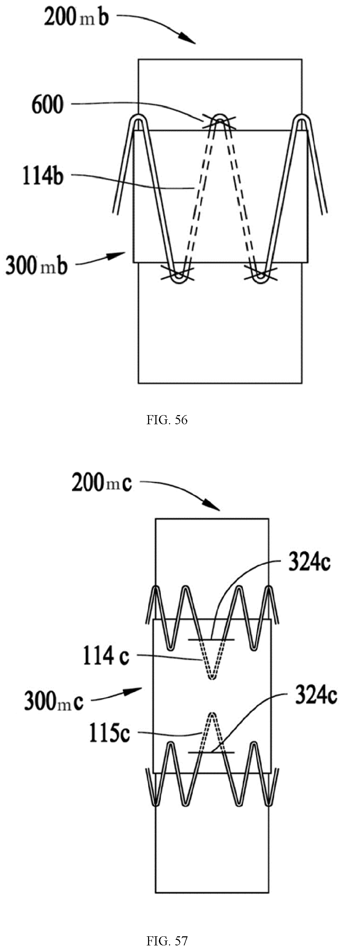

Referring to FIG. 56, FIG. 56 is a structural schematic diagram of the wireless sensor 200mb, the connecting member 300mb and the engagement segment114b in the implant according to another embodiment of the present disclosure. The structures of the sensor 200mb, connecting member 300mb, and engagement segment 114b are essentially the same as those shown in FIG. 16. The difference lies in that the apex and two base ends of the engagement segment 114b are sutured onto the covering membrane of the shunt stent (not shown) via a suturing structure 600.

Referring to FIG. 57, FIG. 57 is a structural schematic diagram of the connecting member and the engagement segment in the implant according to another embodiment of the present disclosure. In this embodiment, the sensor 200mc is encircled by the connecting member 300mc, which is connected to the first engagement segment 114c and the second engagement segment 115c. Specifically, the second connecting portion of the connecting member 300mc is provided with two slit-like openings 324c, which are parallel to the edges of the connecting member 300mc and spaced at a predetermined axial distance. The first engagement segment 114c is a trough portion, while the second engagement segment 115c is a crest portion, arranged opposite each other. The first engagement segment 114c and the second engagement segment 115c are inserted through the two openings 324c into the space between the connecting member 300mc and the sensor 200mc. The respective edges of the two openings 324c and the region between them constitute the clamped portion.

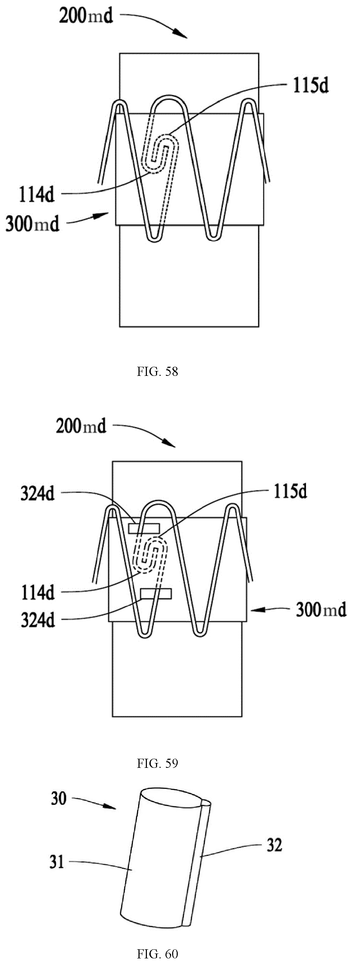

Referring to FIG. 58, FIG. 58 is a structural schematic diagram of the wireless sensor, the connecting member and the engagement segment in the implant according to another embodiment of the present disclosure. The sensor 200md and connecting member 300md in this embodiment are the same with the corresponding structures shown in FIG. 56, with the difference lying in a first engagement segment 114d and a second engagement segment 115d. The first engagement segment 114d and the second engagement segment 115d in this embodiment are similar to the engagement segment structures shown in FIG. 42, but is different from engagement segments of the single-rod, in this embodiment, the elastic curved segments are disconnected at the wave segments, and the terminal ends of the elastic curved segments extend and turn back in their length direction to form U-shaped engagement segments. Moreover, the first engagement segment 114d and the second engagement segment 115d formed by the turned ends overlap in the length direction and are embedded into each other in the radial direction. The specific fabrication process may involve bending two metallic wires separately and embedding them together, or alternatively, during the winding process of a single metallic wire, a portion of the meandering segment may be cut or removed at the middle, causing the meandering segment to form two engagement segments with U-shaped structures.

Referring to FIG. 59, FIG. 59 is a structural schematic diagram of the wireless sensor, the connecting member 300md and the engagement segment in one possible implement. The connection method shown in FIG. 59 is similar to that in FIG. 58, except that the first engagement segment 114d and second engagement segment 115d are not inserted from the edge of the connecting member 300md between the connecting member 300md and the sensor 200md, but rather inserted through two openings 324d provided on the connecting member 300md. Both openings 324d are rectangular and spaced at a predetermined distance axially. In the connection method shown in FIG. 58, the first engagement segment 114d and second engagement segment 115d covered by the connecting member 300md cannot connect with the covering membrane on the stent, resulting in weaker strength at that location. By providing openings 324d on the connecting member 300md, the length of the first engagement segment 114d and second engagement segment 114d overlapped by the connecting member 300md can be reduced, allowing more areas of the engagement segments to connect with the covering membrane and preventing significant weakening of the strength of the stent at that location. Additionally, since the first engagement segment 114d and second engagement segment 114d are U-shaped turned structures, they have a larger area to clamp the connecting member 300md, further enhancing the connection strength with the connecting member. In some possible implementations, adhesive bonding may also be further applied between the first engagement segment 114d and second engagement segment 114d and the connecting member 300md.

Referring to FIG. 60, FIG. 60 is a structural schematic diagram of the connecting member in one possible implement. The connecting member 30 includes a first connecting portion 31 and a second connecting portion 32. In this embodiment, the structure of the connecting member 30 is nearly the same as that of the connecting member 300 shown in FIG. 41, except that an axial dimension of the connecting member 31 in FIG. 60 is larger, enabling it to completely enclose the wireless sensor axially. This configuration ensures a smoother overall surface of the implant, reducing holes or gaps and minimizing the likelihood of thrombus formation. Preferably, the connecting member 30 is made of polymer material, which not only prevents interference with signal transmission but also provides protection for the wireless sensor, avoiding damage to the sensor caused by the stent during delivery.

To better connect the wireless sensor with the stent, the connecting members in the aforementioned embodiments can all be made of heat-shrinkable material. After connecting the engagement segment of the therapeutic device with the connecting member, the heat-shrinking process ensures that the engagement segment is firmly secured between the connecting member and the wireless sensor, thereby achieving a tight connection between the wireless sensor and the stent. In these exemplary embodiments, the engagement segment and the elastic curved segments are integrally formed. However, those skilled in the art will understand that in other modified embodiments, the engagement segment can also be made of a different material from that of the elastic curved segments. For example, the elastic curved segments could be metallic wires, and the engagement segment could be a hard plastic tube or stainless lamellar sheet, etc. The engagement segment and the elastic curved segment can be fixedly connected (e.g., by welding) to achieve the inventive purpose of this application. Nevertheless, from the perspective of processing convenience and usage effectiveness, it is preferable to integrally form the engagement segment with the elastic curved segment. This approach not only ensures a simple, reliable, and compact connection structure but also means that the elastic curved segment itself is part of the stent, eliminating the need for additional structures to connect with the connecting member. This does not significantly increase the radial dimension of the implant and can completely avoid safety issues arising from the connection strength or smoothness between an additional engagement segment and the elastic curved segment.

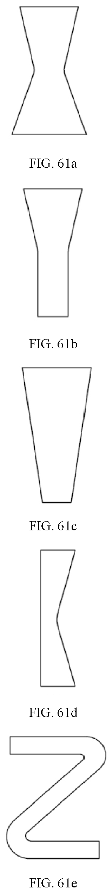

Referring to FIGS. 61a to 61e, some possible implementations of the stent are illustrated.

FIG. 61a is front view of the profile shape of the stent in one possible implement. In this embodiment, the diameter of the middle section of the stent is smaller than that of both ends of the stent, forming a front-view with an X-shaped profile. FIG. 61b is front view of the profile shape of the stent in another possible implement. In this embodiment, the upper section of the stent is flared, while the lower section is straight, forming a front view with a Y-shaped profile. FIG. 61c is front view of the profile shape of the stent in another possible implement. In this embodiment, one end of the stent has a diameter greater than that of another end, with the diameter varying linearly along the axial direction, forming a front view with a V-shaped profile. FIG. 61d is front view of the profile shape of the stent in another possible implement. In this embodiment, the diameters at both ends of the stent differ from that of the middle section of the stent, and the centers of the ends are not coaxial with the center of the middle section, forming the stent a front view with a K-shaped profile. FIG. 61e is front view of the profile shape of the stent in another possible implement. In this embodiment, the stent takes on an overall Z-shape.

The present disclosure also provides an implant system. The implant system comprises a delivery catheter and an implant. The delivery catheter is used to accommodate the compressed implant and deliver the implant to the target site via artificial or natural cavities and/or passages. The implant may include a stent and a wireless sensor, where the stent can be a shunt for atrial septostomy as described in previous embodiments, or it may be an occluder or a vascular stent. The implant is in a compressed state within the delivery catheter. Specifically, the stent is compressed into a C-shaped cross-section, with the outer surface of the stent being compressed and at least partially enclosing the wireless sensor, thereby reducing the diameter of the implant. When the delivery catheter delivers the implant to the target site, the implant can be pushed out of the catheter via an inner catheter.

In some possible embodiments, the implant system further comprises a signal receiving device, which is configured to receive signals from the wireless sensor in the implant, enabling medical personnel to monitor the changes of the physiological parameters of the living body simultaneously.

The foregoing descriptions are merely specific implementations of the present disclosure, but the protection scope of the present disclosure is not limited thereto. Any person skilled in the art can easily conceive of modifications or substitutions within the technical scope disclosed by the present disclosure, and such changes or replacements shall fall within the protection scope of the present disclosure. Therefore, the protection scope of the present disclosure shall be subject to the protection scope of the claims.

## Claims

1. An implant for placement at a target site within a living body, comprising:
a therapeutic device, configured for being placed at the target site to enhance a physiological function of the living body;
a wireless sensor, comprising a monitoring element and a wireless signal transmission element; wherein the monitoring element is configured to acquire target physiological parameters, and the wireless signal transmission element is configured to transmit the target physiological parameters acquired by the monitoring element to an exterior of the living body; and
a connecting member, configured to connect the wireless sensor to the therapeutic device and connected to the wireless sensor in a manner that preserves a signal transmission path of the wireless signal transmission element.

2. The implant according to claim 1, wherein the wireless sensor further comprises a housing, the monitoring element and the wireless signal transmission element are disposed within the housing; the connecting member is connected to the housing in a manner that partially encloses the housing, or in a manner that completely encloses the housing with a non-electromagnetic shielding structure, so as to preserve the wireless signal transmission path of the wireless signal transmission element, and the housing is made of a non-metallic material.

3. The implant according to claim 1, wherein the wireless sensor further comprises a housing, the monitoring element and the wireless signal transmission element are disposed within the housing; the connecting member is provided with an accommodating cavity, the housing of the wireless sensor is provided with a mating structure adapted to the accommodating cavity, and the connecting member is sleeved onto the mating structure via the accommodating cavity.

4. The implant according to claim 1, wherein the connecting member is provided with an insertion portion, and a housing of the wireless sensor is provided with a mating structure adapted to the insertion portion, the insertion portion is inserted into the mating structure, such that the connecting member is connected to a periphery of, or one end of the wireless sensor.

5. The implant according to claim 3 wherein the connecting member is provided with a wireless signal transmission window, when the connecting member is sleeved onto the mating structure of the housing, the wireless signal transmission window is aligned to the wireless signal transmission element to preserve the signal transmission path; the wireless signal transmission element is further configured to receive wireless signals from the exterior of the living body.

6. The implant according to claim 3 or 4, wherein the connecting member comprises a hanging rod and a clamping ring, the clamping ring is sleeved onto the mating structure, one end of the hanging rod is connected to the clamping ring, and another end of the hanging rod is connected to the therapeutic device.

7. The implant according to claim 6, wherein the accommodating cavity is provided on the clamping ring, the mating structure is a groove, an extending direction of the hanging rod is parallel to a central axis of the clamping ring; the connecting member further comprises an upper cover or a lower cover, and the upper cover or the lower cover is integrally formed with the clamping ring and is sleeved on one end of the housing.

8. The implant according to claim 6 or 7, wherein the clamping ring is misaligned to the wireless signal transmission element.

9. The implant according to claim 6 or 7, wherein the clamping ring is aligned to the wireless signal transmission element, and the clamping ring is made of a non-metallic material.

10. The implant according to claim 6 or 7, wherein an opening is provided on a side wall of the clamping ring.

11. The implant according to claim 6 or 7, wherein the clamping ring is a metallic tubular structure formed by helically winding a metallic strip.

12. The implant according to any one of claims 6 to 11, wherein the clamping ring comprises an opening and reinforcement holes, the reinforcement holes are disposed at both ends of the clamping ring near the opening, for threading reinforcement members.

13. The implant according to any one of claims 6 to 12, wherein a groove is provided on the surface of the housing, the groove is adapted to the shape of the clamping ring, and the clamping ring is embedded in the groove.

14. The implant according to any one of claims 6 to 13, wherein the therapeutic device comprises a mesh stent formed by cutting, the hanging rod, the clamping ring and the mesh stent are integrally formed by cutting.

15. The implant according to any one of claims 3 to 14, wherein the wireless sensor is hooked to the therapeutic device via the connecting member.

16. The implant according to claim 15, wherein the therapeutic device is a valve stent, and the wireless sensor is fixed to an inflow end or an outflow end of the valve stent via the connecting member.

17. The implant according to claim 15, wherein the connecting member is connected to a wave rod of the valve stent.

18. The implant according to claim 3 or 4, wherein when a portion of the connecting member is sleeved onto or is inserted into the mating structure, another portion of the connecting member is sleeved on one end of the therapeutic device.

19. The implant according to claim 3 or 4, wherein when a portion of the connecting member is inserted into the mating structure, another portion of the connecting member is inserted into the therapeutic device.

20. The implant according to claim 18 or 19, wherein the portion of the connecting member connected to the mating structure of the housing is an unclosed sleeve.

21. The implant according to claim 18 or 19, wherein the therapeutic device is an occluder or a valve clip, the valve clip comprises a body portion, one end of the wireless sensor is inserted into the body portion via the connecting member, and the wireless signal transmission element is located outside the body portion.

22. The implant according to claim 18 or 19, wherein the therapeutic device is an annuloplasty ring or a leadless pacemaker, and the wireless sensor is sleeved onto one end of the annuloplasty ring or the leadless pacemaker via the connecting member.

23. The implant according to claim 3, wherein when the connecting member is sleeved onto the housing of the wireless sensor, the connecting member is configured to surround a periphery of the wireless sensor, and the therapeutic device is connected to the periphery of the wireless sensor via a lamellar structure or a filamentous structure inserted between the connecting member and the housing.

24. The implant according to claim 23, wherein the number of the lamellar structures or of the filamentous structures is two, and the two lamellar structures or the two filamentous structures are respectively inserted between the connecting member and the housing from opposite directions.

25. The implant according to claim 23 or 24, wherein the connecting member comprises at least one sleeve, the sleeve is provided with an opening, the opening is configured to allow the lamellar structure or the filamentous structure to enter between the sleeve and the wireless sensor.

26. The implant according to any one of claims 23 to 25, wherein the connecting member comprises at least one sleeve, the sleeve is formed by clasping two semi-rings.

27. The implant according to any one of claims 23 to 26, wherein the connecting member is a polymer sleeve, the lamellar structure or the filamentous structures is inserted between a wall of the sleeve and the wireless sensor, and is clamped by the polymer sleeve.

28. The implant according to any one of claims 23 to 27, wherein the therapeutic device comprises a stent formed by braiding, winding or cutting, and the stent is connected to the wireless sensor via the lamellar structure or the filamentous structure.

29. The implant according to claim 28, wherein when the stent is connected to the wireless sensor via the filamentous structure, the filamentous structure is integrally formed with a body portion of the stent.

30. The implant according to any one of claims 23 to 27, wherein the therapeutic device is a chordae tendineae repair element, the chordae tendineae repair element comprises an artificial chordae tendineae, and the connecting member clamps the the artificial chordae tendineae onto the surface of the wireless sensor.

31. The implant according to claim 3, wherein the connecting member comprises an insertion rod provided on a surface of the housing and a sleeve, the sleeve is sleeved onto the filamentous structure of the therapeutic device, and the insertion rod is inserted between the filamentous structure and the sleeve.

32. The implant according to claim 1, wherein the connecting member is a through-hole provided on a wall of the housing and extending parallel to an axial direction, a filamentous structure is inserted into the through-hole, such that the therapeutic device is connected to the wireless sensor.

33. The implant according to claim 32, wherein the number of through-holes is at least two, and the filamentous structure connects the wireless sensor to the therapeutic device by sequentially passing through at least two of the through-holes.

34. The implant according to claim 32 or 33, wherein the therapeutic device is a cardiac catheter pump assembly, the cardiac catheter pump assembly comprises a guide wire, the guide wire forms the filamentous structure.

35. The implant according to claim 1, wherein a connection between the connecting member and the therapeutic device is a mortise-and-tenon joint, a housing of the wireless sensor and the connecting member are of an integrated structure, the connecting member is provided on the housing and comprises a corresponding interface for forming a concave-convex mating connection with the therapeutic device.

36. The implant according to claim 35, wherein the corresponding interface is a protrusion or a recess provided on a peripheral surface of the housing.

37. The implant according to claim 35 or 36, wherein the corresponding interface is an internal thread, an external thread, or a spring provided at one end of the housing.

38. The implant according to claim 1, wherein the therapeutic device is a stent, the stent comprises a body formed by a plurality of elastic curved segments and at least one engagement segment, the engagement segment is disposed at a predetermined region of the body and connected to a portion of at least one of the elastic curved segments; and
the connecting member comprises a first connecting portion and a second connecting portion that are interconnected, the first connecting portion is connected to the wireless sensor, and the second connecting portion is connected to the engagement segment, thereby enabling the wireless sensor to be attached to the stent at the predetermined region.

39. The implant according to claim 38, wherein the second connecting portion comprises a clamped portion, and the engagement segment is inserted into the second connecting portion to clamp the clamped portion; and
the engagement segment is integrally formed with the portion of at least one elastic curved segment, and the engagement segment forces the clamped portion to abut against the peripheral surface of the body via its own elastic force.

40. The implant according to claim 39, wherein the clamped portion abuts against the peripheral surface of the body by the engagement segment with an adhesive.

41. The implant according to any one of claims 38 to 40, wherein the stent and the wireless sensor are arranged side by side, and the connecting member attaches the wireless sensor to the peripheral surface of the stent in the predetermined region.

42. The implant according to claim 38 or 41, wherein the second connecting portion comprises a sheet portion, the sheet portion comprises an edge portion, the edge portion forms the clamped portion, and the engagement segment is connected to the sheet portion by clamping the edge portion.

43. The implant according to claim 42, wherein the edge portion is located at an edge region of the sheet portion or at an edge region of an opening formed on the sheet portion.

44. The implant according to claim 42, wherein the connecting member is an annular sleeve, a part of the annular sleeve serves as the first connecting portion, and another part of the annular sleeve serves as the second connecting portion; and the edge portion of the sheet portion is formed by an edge portion of the annular sleeve.

45. The implant according to claim 42, wherein the connecting member is an annular sleeve, a part of the annular sleeve serves as the first connecting portion, and another part of the annular sleeve serves as the second connecting portion; and an opening is provided on the annular sleeve, the edge portion of the sheet portion is formed by an edge portion of the opening.

46. The implant according to claim 44 or 45, wherein the annular sleeve at least partially overlaps with the wireless signal transmission element in an axial direction; and the annular sleeve is a non-metallic sleeve, and a cross-section of the non-metallic sleeve is an enclosed structure or an unclosed structure.

47. The implant according to claim 44 or 45, wherein the annular sleeve at least partially overlaps with the wireless signal transmission element in an axial direction; and the annular sleeve is a metallic sleeve, and a cross-section of the metallic sleeve is an unclosed structure.

48. The implant according to claim 44 or 45, wherein the annular sleeve completely overlaps the wireless sensor in an axial direction, with the monitoring element being exposed on the end face of the wireless sensor, the monitoring element is configured to monitor pressure.

49. The implant according to any one of claims 38 to 48, wherein the elastic curved segment forms the body by extending circumferentially and axially, and the elastic curved segment comprises at least a wave segment, a portion of the wave segment forms the engagement segment.

50. The implant according to claim 49, wherein a predetermined trough portion and/or a predetermined crest portion of the wavy segment forms the engagement segment.

51. The implant according to any one of claims 38 to 40, wherein the engagement segment comprises a first engagement segment and a second engagement segment, the second connecting portion comprises a clamped portion, and the first engagement segment and the second engagement segment respectively clamp the clamped portion from two opposite directions.

52. The implant according to claim 51, wherein the elastic curved segment forms the body by extending circumferentially and axially, and the elastic curved segment at least comprises a wave segment, a portion of the wave segment forms the first engagement segment and the second engagement segment, the first engagement segment and the second engagement segment comprise bent and turned ends, and the bent and turned ends of the first engagement segment and the second engagement segment are nested with each other.

53. The implant according to claim 52, wherein the connecting member is an annular sleeve, one portion of the annular sleeve serves as the first connection part and another portion of the annular sleeve serves as the second connection part; and the annular sleeve is provided with a first opening and a second opening spaced apart axially by a predetermined distance, and the first engagement segment and the second engagement segment are inserted through the first opening and the second opening, respectively, between the annular sleeve and the wireless sensor.

54. The implant according to any one of claims 38 to 53, wherein the stent is an atrial shunt device, the body is a tubular body for shunting, and the tubular body is formed by the elastic curved segments extending in a circular and/or helical pattern.

55. The implant according to claim 54, wherein the tubular body is configured to be radially resiliently deformable while maintaining a constant perimeter.

56. The implant according to claim 54 or 55, wherein the tubular body is configured to be resiliently compressible along a first radial direction within a confined space.

57. The implant according to any one of claims 54 to 56, wherein the tubular body is configured to have a C-shaped cross-sectional profile when compressed along a first radial direction within a confined space.

58. The implant according to any one of claims 54 to 57, wherein the wireless sensor is parallel to the body.

59. The implant according to any one of claims 54 to 58, wherein the wireless sensor is connected to the body tangentially and externally.

60. The implant according to any one of claims 38 to 59, wherein the stent further comprises a clamping arm group, the clamping arm group comprises at least a first clamping arm and a second clamping arm, the first clamping arm and the second clamping arm are axially spaced apart along the body and distributed around the periphery of the body, the first clamping arm and the second clamping arm each forms a cantilever protruding from the periphery of the body.

61. The implant according to claim 60, wherein the first clamping arm and/or the second clamping arm is formed by bending an elastic metallic wire.

62. The implant according to claim 60 or 61, wherein the first clamping arm and/or the second clamping arm is provided with a U-shaped or V-shaped structure formed by extending the elastic metallic wire radially for a predetermined distance and then turning back.

63. The implant according to claim 61 or 62, wherein an elastic wire constituting the first clamping arm and/or the second clamping arm and an elastic wire of the tubular body to which the base of the first clamping arm and/or the second clamping arm is attached are the same elastic wire.

64. The implant according to any one of claims 61 to 63, wherein the first clamping arm and/or the second clamping arm have a predetermined distance from the respective adjacent end of the tubular body.

65. The implant according to any one of claims 60 to 64, wherein a free end of the first clamping arm overlaps with a free end of the second clamping arm in an axial projection, and a fixed end of the first clamping arm is staggered with a fixed end of the second clamping arm in the axial projection.

66. The implant according to any one of claims 38 to 65, wherein the stent further comprises a covering membrane provided within the body.

67. The implant according to any one of claims 38 to 65, wherein the stent further comprises a covering membrane, the covering membrane is provided outside the body, and the covering membrane is provided with a gap exposing the engagement segment.

68. The implant according to any one of claims 38 to 67, wherein the connecting member is made of polymer material and connects the wireless sensor to the stent via a heat-shrink process.

69. An implant system comprising a delivery catheter and an implant according to any one of claims 38 to 68, wherein the implant is compressed when being disposed within the delivery catheter, the stent is compressed into a C-shaped cross-sectional structure, an outer surface of the compressed stent at least partially surrounds the wireless sensor, and the implant is capable of being out from the catheter.

70. The implant system according to claim 69, further comprising a signal receiving device, wherein the signal receiving device is configured to receive signals from the wireless sensor in the implant.
